# EUROPÄISCHE PATENTSCHRIFT

(11) **EP 3 039 118 B1**
(45) Veröffentlichungstag und Bekanntmachung des Hinweises auf die Patenterteilung: **06.03.2019**
(21) Anmeldenummer: 14796677.4
(22) Anmeldetag: 29.08.2014
(51) Int. Cl.: C12M 1/32, C12M 1/04, C12M 1/00, C12M 1/12, C12M 1/34, C12Q 1/02, G01N 33/50

(54) **EXPOSITIONSAPPARAT**
EXPOSURE APPARATUS
APPAREIL D'EXPOSITION

(30) Priorität: 30.08.2013 DE 102013109450
(43) Veröffentlichungstag der Anmeldung: 06.07.2016
(73) Patentinhaber: Fraunhofer Gesellschaft zur Förderung der angewandten Forschung E.V., 80686 München (DE)
(72) Erfinder: RITTER, Detlef, 30519 Hannover (DE); KNEBEL, Jan, 30625 Hannover (DE)
(74) Vertreter: Patentanwälte Thömen & Körner
(86) Internationale Anmeldenummer: PCT/DE2014/100312
(87) Internationale Veröffentlichungsnummer: WO 2015/027998

(56) Entgegenhaltungen:
- WO-A2-02/24861
- DE-A1-102007 030 413
- DE-A1-102009 039 868
- US-A- 5 863 792
- US-A1- 2005 260 745
- US-A1- 2010 009 335
- US-A1- 2010 112 690
- DETLEF RITTER ET AL: "Investigations of the Biological Effects of Airborne and Inhalable Substances by Cell-Based In Vitro Methods: Fundamental Improvements to the ALI Concept", ADVANCES IN TOXICOLOGY, Bd. 26, Nr. 3, 12. November 2014 (2014-11-12), Seiten 187-11, XP055162030, ISSN: 2356-6906, DOI: 10.1098/rsta.2011.0300

## Beschreibung

Die Erfindung betrifft einen Expositionsapparat zur Durchführung von in-vitro-Experimenten mit technischen Replikaten wenigstens eines biologischen Testsystems, die perfusiv unter Ausbildung einer Expositionsatmosphäre auf bzw. oberhalb des biologischen Testsystems mit einem Expositionsmedium beaufschlagbar sind.

Bei der Durchführung von in-vitro-Experimenten mit biologischen Testsystemen, insbesondere mit Zellen, Zellkulturen oder Geweben, wird üblicherweise - unabhängig von der Zielsetzung der Experimente bzw. der Untersuchungen - eine Substanz in Kontakt mit dem biologischen Testsystem gebracht, so genannte Exposition.

Das biologische Testsystem bzw. biologische Prüfsystem wird hierbei einer Expositionsatmosphäre, die durch ein Expositionsmedium gebildet wird, ausgesetzt. Das Expositionsmedium kann die Substanz, die auch als Test- oder Prüfsubstanz bezeichnet wird, enthalten und/oder diese bilden. In einigen Fällen kann es wünschenswert sein, wenn das Expositionsmedium ohne Substanz bzw. Test- oder Prüfsubstanz ausgebildet ist.

Bei dem biologischen Testsystem kann es sich um eine Eukaryonten-Kultur, besonders bevorzugt um Zelllinien, Primärzellisolate, Gewebeschnitte, rekonstruierte Gewebe wie Kokulturen, oder genetisch veränderte Zellen, oder eine Prokaryonten-Kultur handeln.

Das Expositionsmedium kann flüssig oder gasförmig sein.

Das gasförmige Expositionsmedium kann als ein reines Gas oder Gasgemisch vorliegen, d. h. alle darin enthaltenen Stoffe, insbesondere Atome, Moleküle, etc., befinden sich in der Gasphase, und/oder es kann auch als Träger für Fest- und/oder Flüssigstoffe dienen. Insbesondere können so Aerosole, zerstäubte Flüssigkeiten, kleine Flüssigkeitströpfchen (beispielsweise Pflanzenschutzmittel als Sprühnebel, etc.), Schwebeteilchen, Feststoffpartikel (beispielsweise Holzstaub, etc.), gasförmige Suspensionen, zerstäubte Suspensionen oder Emulsionen als zu tragende Substanzen in dem Trägergas enthalten sein.

Bei den genannten in-vitro-Experimenten kann es sich um gezielte Manipulationen durch pharmakologisch wirksame Substanzen handeln. Es kann sich bei den genannten in-vitro-Experimenten aber auch um solche handeln, die beispielsweise im Rahmen von so genannten Tissue-Engineering-Techniken Einfluss auf Zellwachstum oder -differenzierung nehmen sollen. Auch kann es sich bei den genannten in-vitro-Experimenten um toxikologische Untersuchungen unterschiedlichster Substanzen bzw. Stoffe handeln.

Hinsichtlich der Art und Weise, wie die Substanzen mit den biologischen Testsystemen in Kontakt gebracht werden, kann man grundsätzlich zwei Arbeitsweisen unterscheiden, nämlich zum einen die statische Exposition und zum anderen die dynamische Exposition, die auch als Perfusion bzw. perfusive Exposition bezeichnet wird.

Im Falle der statischen Exposition wird die Substanz einmalig oder auch wiederholt, insbesondere additiv, aber nicht kontinuierlich zum biologischen Testsystem gegeben, auf dem sie dann für einen gewissen Zeitraum verbleibt, ohne ausgetauscht zu werden.

Im Falle der dynamischen Exposition wird die Substanz kontinuierlich zu- und abgeleitet, etwa um eine höhere Effektivität oder Sensitivität in der Untersuchung zu erzeugen, oder auch aufgrund physikalisch/chemischer oder anderer Eigenschaften der Testsubstanzen, des biologischen Testsystems oder anderer Randbedingungen der Untersuchung.

Möglichkeiten zur Durchführung statischer Expositionen sind in vielfältiger Weise verwirklicht und in breiter Auswahl kommerziell erhältlich. Üblicherweise wird dabei die in einem Puffer oder Kulturmedium aufgenommene, insbesondere suspendierte oder gelöste Substanz in einen Well bzw. eine Vertiefung bzw. Kavität einer Multiwell-Platte gegeben, in welcher sich auch das biologische Testsystem befindet.

Möglichkeiten zur Durchführung dynamischer Expositionen sind dagegen weniger vielfältig vorhanden.

Zur Durchführung von dynamischen Expositionen bzw. Perfusionsexpositionen mit flüssigen Expositionsmedien sind Vorrichtungen beispielsweise in der WO 2005/123950 A2 bzw. in der US 2005/260745 A1, in der DE 33 17 551 A1 oder auch von Domansky et al. in Lab Chip (2010), 10, 51-58, offenbart.

Zur Durchführung von dynamischen Expositionen bzw. Perfusionsexpositionen mit gasförmigen Expositionsmedien sind Vorrichtungen in der DE 195 26 533 A1, der US 2010/0273246 A1, der WO 2010/040473 A2 oder die auf die Erfinder der vorliegenden Patentanmeldung zurückgehenden DE 10 2007 030 413 A1 sowie DE 10 2009 039 868 A1 offenbart.

Allen diesen Vorrichtungen ist gemein, dass sie Speziallösungen darstellen, die mit der üblichen Standardausrüstung eines Labors nicht ohne weiteres kompatibel sind und sich daher nicht zeit- und kostensparend in die etablierten Arbeitsabläufe integrieren lassen.

Darüber hinaus haben alle Vorrichtungen zur Durchführung von dynamischen Expositionen bzw. Perfusionsexpositionen mit gasförmigen Expositionsmedien - mit Ausnahme der in der DE 10 2007 030 413 A1 und der DE 10 2009 039 868 A1 offenbarten Vorrichtungen - den Nachteil, dass sie technisch eine Vermischung der zwei vorhandenen Fluidphasen zulassen.

Bei den Fluidphasen handelt es sich nach dem Stand der Technik auf der einen Seite um die flüssige Phase in Form des Erhaltungsmediums bzw. Nährmediums, das von der Unterseite an die das biologische Testsystem enthaltenen Kulturgefäße zur Erhaltung des biologischen Testsystems herangeführt wird, und auf der anderen Seite um die gasförmige Phase in Form des Expositionsmediums bzw. der Expositionsatmosphäre, in der die Substanz bzw. die Test- oder Prüfsubstanz gasförmig bzw. Luft getragen an das biologische Testsystem herangeführt wird. Eine Vermischung hat in der Untersuchung vielfältige nachteilige Konsequenzen, die in einem Mangel an Reproduzierbarkeit, Erzeugen unerwünschter Nebenprodukte und einer ungenügend definierte (Prüf-)Situation des biologischen Testsystems resultieren.

Unter einem Erhaltungsmedium im Sinne der Erfindung wird hier und im Folgenden ein Kulturmedium, auch als Nährmedium bezeichnet, mit oder ohne Zusätzen oder eine Salzlösung verstanden.

Neben den genannten Vorrichtungen zur Durchführung von dynamischen Expositionen bzw. Perfusionsexpositionen sind Vorrichtungen zur gezielten Zellkultivierung bekannt. Mit solchen Vorrichtungen sollen sich vorzugsweise automatisiert die für das Wachstum der Zellen förderlichen Kulturbedingungen einstellen lassen.

Aus der WO 02/24861 A2 ist beispielsweise eine Vorrichtung zum automatisierten Züchten und/oder Behandeln von Zellen für diagnostische Zwecke bekannt, welche eine Multiwellplatte mit einer Vielzahl von Wells aufweist, in denen Zellen aufgenommen werden. Die Wells sollen hierbei von der Oberseite her von Nährstoffen und/oder Sauerstoff durchströmt werden, die das Wachstum der Zellen fördern. In den Wells selbst sind keine separaten Kulturgefäße, so genannte Inserts, aufgenommen.

Für das Durchströmen ist in jedem Well ein Einsatz eingebracht, der eine Zulaufbohrung und eine Rücklaufbohrung aufweist. Der Einsatz weist auf der zum Boden des Wells gerichteten Seite einen Trennsteg zwischen der Zulaufbohrung und der Rücklaufbohrung auf, damit die über die Zulaufbohrung eingebrachten Nährstoffe nicht nach Art eines Kurzschlusses direkt zur Rücklaufbohrung strömen können. Zur Abdichtung der Wells ist jeder Einsatz umfangseitig mit einem Dichtring versehen.

Anstelle der in die Wells einbringbaren Einsätze kann auch eine Deckplatte mit darin eingesetzten Zulauf- und Rücklaufanschlüssen vorgesehen sein, wobei jedem Well ein Zulauf- und ein Rücklaufanschluss zugeordnet sind. Der Zulaufanschluss ragt dabei tiefer in das Well als der Rücklaufanschluss, um auch hier den vorher erwähnten Kurzschluss zu vermeiden. Die Deckplatte kann durch Schrauben mit der Zellkulturplatte verbunden sein. Anstelle oder zusätzlich zu dieser Verbindung kann auch eine Abdichtung durch eine Dichtungseinrichtung zwischen Deckplatte und Zellkulturplatte erreicht werden.

Die Deckplatte kann gemäß WO 02/24861 A2 zweiteilig mit einer oberen und unteren Abdeckung ausgebildet sein, wobei die obere und die untere Abdeckung abstandseinstellbar miteinander verbunden sind. Durch dazwischen angeordnete Federn kann eine Vorspannung erreicht werden. Die obere Abdeckung wird über Stege an den Rändern der Zellkulturplatte umlaufend auf diese aufgesetzt. Durch die untere Abdeckung sind die Zulaufanschlüsse und Rücklaufanschlüsse geschoben, wobei diese entsprechend auch durch die obere Abdeckung entweder auf deren Oberseite oder gegebenenfalls auch seitlich herausgeführt sind. Durch die Federn soll eine Abdichtung der Wells erreicht, dadurch dass die untere Abdeckung entsprechend dicht auf der Oberseite der Zellkulturplatte gedrückt wird. Gleichzeitig werden auf diese Weise auch Ansätze, die auf der Unterseite der unteren Abdeckung nach unten aus dieser vorstehen in die Wells zur Bildung von abgeschlossenen Zellkulturräumen eingeführt. Zum einen liegt die untere Abdeckung also auf den Rändern der Wells auf und zum anderen ist sie über die Ansätze in den Wells eingebracht.

Aus der US 8,501,462 B2 bzw. US 2010/112690 A1 ist eine Anordnung aus einer Multiwellplatte und einer mehrere Einsätze aufweisenden Einsatzplatte bekannt. Multiwellplatte und Einsatzplatte sind miteinander verbindbar, wobei jeder Einsatz der Einsatzplatte in einem Well der Multiwellplatte positionierbar ist. Die Einsatzplatte liegt hierbei der Multiwellplatte auf.

Jeder Einsatz weist einen Zufuhranschluss, der mit einer Gasversorgungsquelle verbindbar ist, und einen Auslassanschluss aus. Mehrere Zufuhranschlüsse können über einen Gasverteiler mit der Gasversorgungsquelle verbindbar sein. Mehrere Zufuhranschlüsse können entsprechend eine gemeinsame Gasversorgungsleitung aufweisen. Mehre Auslassanschlüsse können an einer gemeinsamen Gasauslassleitung angeschlossen sein. Am Boden jedes Einsatzes ist von unten her eine gasdurchlässige Membran in einem vorgegebenen Abstand vom Boden des Einsatzes befestigt, wodurch zwischen Boden und gasdurchlässiger Membran ein Strömungskanal ausgebildet ist, der eine Strömungsverbindung zwischen dem Zufuhranschluss und dem Auslassanschluss bildet. Zwischen Boden und gasdurchlässiger Membran können auch mehrere den Einlassanschluss und Auslassanschluss verbindbare Strömungskanäle ausgebildet sein, wenn die gasdurchlässige Membran mehrere nach oben gerichtete Vorsprünge aufweist, deren Oberseiten mit dem Boden des Einsatzes verbunden sind. Die Anzahl, Anordnung und das Ausmaß der Vorsprünge bestimmen den Verlauf des oder der Strömungskanäle sowie den Abstand der Membran vom Boden des Einsatzes.

Jede gasdurchlässige Membran des in einem Well angeordneten Einsatzes ist zudem in einem vorgegebenen Abstand von der der Grundfläche des Wells angeordnet. Jeder Einsatz kann zudem einen separaten Medieneinlass und Medienauslass aufweisen, die miteinander in Strömungsverbindung stehen, wobei sich ein Strömungskanal zwischen gasdurchlässiger Membran des Einsatzes und Grundfläche des Wells ausbildet. Für die vorgesehenen Anwendungsfälle werden Zellen auf der Grundfläche eines Wells gezüchtet und mit einem das Wachstum der Zellen beeinflussenden Gas, welches durch die gasdurchlässige Membran des Einsatzes diffundiert, in Kontakt gebracht. Die Anordnung befindet hierzu in einem Inkubator.

US 5,863,792 offenbart eine Vorrichtung zum Züchten von Zellen oder Gewebekulturen in vitro. Die Vorrichtung weist eine Multiwellplatte sowie einen abnehmbaren Deckel auf. Der Deckel dient dazu, den Verlust von Zellen oder Proben aus den Wells der Multiwellplatte zu verhindern, den Inhalt der Multiwellplatte vor der Umgebung zu schützen und den Anwender vor dem Inhalt der Multiwellplatte zu schützen, falls diese ein schädliches oder potentiell schädliches Material enthalten sollte. Zudem sind Mittel zum abnehmbaren Befestigen des Deckels an der Platte vorgesehen, wodurch ein längs des inneren Randumfangs des Deckels befindliches Dichtungsmaterial zwischen Deckel und Multiwellplatte zusammengedrückt wird und das Innere der Multiwellplatte vor der äußeren Umgebung geschützt wird, wodurch in der Multiwellplatte eine biologisch sichere Umgebung bereitgestellt wird.

US 2010/0009335 A1 offenbart eine Vorrichtung für die in-vitro Kultivierung von Zellen oder Gewebekulturen in einem Wachstumsmedium. Die Vorrichtung umfasst eine Multiwellplatte, in deren Wells Zellen oder Gewebe kultiviert werden, wobei Einrichtungen vorgesehen sind, um die Wells der Vorrichtung gegenüber der Umwelt zu isolieren, um die Temperatur des Wachstumsmediums zu steuern, um die Sicht auf die Zellen oder die Gewebekultur innerhalb eines Wells und die Steuerung oder Regulierung der Wachstumsbedingungen innerhalb eines Wells zu erhalten, einschließlich der gesteuerten Regulierung und Überwachung beispielsweise der in das und aus dem Well geführten Medium-Gase, wie O2, CO2 oder N2, des dort vorliegenden pH-Werts oder der dort herrschenden Temperatur.

Die Multiwellplatte umfasst ein Mikro-Heizelement, das derart ausgebildet ist, dass es die Temperatur eines oder mehrerer Wells bzw. eines Wachstumsmediums zur Optimierung der Wachstumsbedingungen steuert, wodurch auf einen separaten Inkubator verzichtet werden kann.

Die Vorrichtung umfasst auch einen auf die Multiwellplatte aufsetzbaren Deckel, wobei ein weiteres Mikro-Heizelement vorgesehen sein kann, um die Temperatur des Deckels zu steuern. Die Temperatur des Deckels soll dabei höher als die Temperatur des Wells sein, um eine Kondensation auf dem Deckel zu verhindern.

Zwischen Deckel und jedem Well kann eine oder mehr als eine Dichtung, vorteilhaft in Form eines O-Rings, vorgesehen sein, um den Rand der Wells abzudichten und einen unkontrollierten Materialaustausch zwischen den Wells zu verhindern. Die Dichtung soll auch dazu beitragen, die innerhalb jedes Wells gebildete Mikroumgebung aufrechtzuerhalten bzw. eine biologische Isolierung jedes Wells zu erreichen.

Der Deckel kann auf den Wells durch eine Klick- oder Klemmverbindung befestigt werden und die zuvor genannten Einrichtungen aufweisen.

Die Vorrichtung kann weiterhin eine Vielzahl von Ports aufweisen, die mit einem oder mehreren Wells in Verbindung stehen. Zumindest einige der Ports können als Septum-Ports ausgebildet sein, um eine Probenentnahme ohne Aufhebung der Dichtung bzw. der biologischen Isolierung eines Wells zu ermöglichen.

Weiterhin können die Ports so ausgelegt sein, dass über diese Medien, beispielsweise Gas oder ein Nährmedium, den im Well befindlichen Zellen oder Gewebekulturen zuführbar ist. Steuereinrichtungen können vorhanden sein, um Fluide, beispielsweise Gase oder Nährmedien, in die und verbrauchte Fluide aus den Wells zu leiten.

Auch die vorgenannten Vorrichtungen stellen Speziallösungen dar, die mit der üblichen Standardausrüstung eines Labors, insbesondere mit Multiwellplatten, in deren Wells Kulturgefäße eingehängt sind, nicht ohne weiteres kompatibel sind und sich daher nicht allumfassend und damit zeit- und kostensparend in die etablierten Arbeitsabläufe integrieren lassen.

Ausgehend von diesem Stand der Technik liegt der Erfindung die Aufgabe zugrunde, einen Expositionsapparat zur Durchführung von in-vitro-Experimenten mit technischen Replikaten wenigstens eines biologischen Testsystems, welche perfusiv unter Ausbildung einer Expositionsatmosphäre mit einem Expositionsmedium beaufschlagbar sind, bereitzustellen, der eine Alternative zu den genannten Speziallösungen, die mit der üblichen Standardausrüstung eines Labors nicht ohne weiteres kompatibel sind und sich daher nicht zeit- und kostensparend in die etablierten Arbeitsabläufe integrieren lassen, bildet. Zudem soll der entsprechende Expositionsapparat eine hohe Reproduzierbarkeit und Effektivität der Funktionalität sicherstellen, die insbesondere eine individuelle Beaufschlagung der biologischen Testsysteme ermöglichen soll. Darüber hinaus soll ein entsprechender Expositionsapparat bereitgestellt werden, bei welchen ein Vermischen von Fluidphasen, insbesondere von Erhaltungsmedium und Expositionsmedium, unterbunden wird.

Diese Aufgabe wird durch einen Expositionsapparat mit den Merkmalen des Anspruchs 1 gelöst. Weiterbildungen und vorteilhafte Ausgestaltungen der Erfindung ergeben sich aus den Unteransprüchen.

Der erfindungsgemäße Expositionsapparat zur Durchführung von in-vitro-Experimenten mit technischen Replikaten wenigstens eines biologischen Testsystems, die perfusiv unter Ausbildung einer Expositionsatmosphäre oberhalb des biologischen Testsystems mit einem Expositionsmedium beaufschlagbar sind, umfasst
- einen Expositionsaufsatz, der derart dimensioniert ist, dass dieser auf eine Standard-Multiwellplatte aufsetzbar und mit dieser mittels geeigneter Verbindungselemente verbindbar ist, wobei der Expositionsaufsatz mehrere Leitungselemente mit jeweils zumindest einer Zuleitung und zumindest einer Ableitung für ein Expositionsmedium aufweist
- wobei jedes Leitungselement aus der zur Standard-Multiwellplatte weisenden Seite des Expositionsaufsatzes hervorragt und einem bestimmten, vorzugsweise mit biologischem Testsystem versehenen Well der Standard-Multiwellplatte zur Ausbildung der Expositionsatmosphäre innerhalb des bestimmten Wells zugeordnet ist,
- wobei die Wells Aufnahmen sind, die bereits in der Standard-Multiwellplatte integriert sind, oder solche, die als separate Kulturgefäße, so genannte Inserts, in vorhandene integrierte Wells oder in entsprechend angeordnete Aussparungen der Standard-Multiwellplatte aufgenommen sind,
- wobei der Expositionsaufsatz (16) einen auf die Standard-Multiwellplatte (18) aufsetzbaren und mit dieser mittels der Verbindungselemente (36) verbindbaren Aufsatzhalterahmen (34) sowie einen bewegbaren auf dem Aufsatzhalterahmen (34) angeordneten und mit diesem (34) verbundenen Aufsatzkopf (44) aufweist,
- wobei das jeweils einem bestimmten Well (20) zugeordnete Leitungselement (22) nach dem Aufsetzen des Expositionsaufsatzes (16) auf die Standard-Multiwellplatte (18) durch Bewegen des mit den Leitungselementen (22) zusammenwirkenden Aufsatzkopfes (44) in Richtung Aufsatzhalterahmen (34) und Standard-Multiwellplatte (18) in das bestimmte Well (20) bewegbar und derart einbringbar ist, dass ein auf der Außenseite (26) des Leitungselements (22) vorgesehenes und dieses umlaufende Dichtmittel (28) das Leitungselement (22) gegenüber der Innenwand (30) des Wells (20) zur Ausbildung einer während des in-vitro-Experiments innerhalb jedes bestimmten Wells (20) abgeschlossenen Expositionsatmosphäre (14) dicht abschließt,
- wobei nur über das jeweils zugeordnete Leitungselement (22) Expositionsmedium (14) zu- und ableitbar ist, wobei der Aufsatzkopf (14) mittels vorzugsweise am Aufsatzkopf (44) angeordneter Ver- und Entriegelungselemente (46) mit dem Aufsatzhalterahmen (34) zur oder während der Durchführung des Experiments derart verriegelbar ist, dass die Leitungselemente (22) festgelegt sind, wobei die Verriegelungselemente (46) den Aufsatzkopf (44) und den Aufsatzhalterahmen (34) vorzugsweise unter Spannung zusammenhalten und wobei der Aufsatzkopf (44) nach der Durchführung des Experiments mittels der Ver- und Entriegelungselemente (46) von dem Aufsatzhalterahmen (34) entriegelbar ist, derart, dass die Leitungselemente (22) samt umlaufende Dichtmittel (28) mit Bewegen des Aufsatzkopfes (44) in entgegengesetzter Richtung zur vorherigen aus den Wells (20) lösbar sind.

Mit dem erfindungsgemäßen Expositionsapparat sind erstmals in-vitro-Experimente mit technischen Replikaten wenigstens eines biologischen Testsystems, welche perfusiv unter Ausbildung einer Expositionsatmosphäre mit einem Expositionsmedium beaufschlagbar sind, unter Verwendung von Standard-Multiwellplatten möglich, die eine Anzahl in Positionierung und Dimensionierung genormter Wells, die auch als Vertiefungen oder Kavitäten bezeichnet werden, besitzen und es dadurch ermöglichen, in Zeit und Kosten schonender Arbeitsweise eine große Zahl von Untersuchungen unter hohen Qualitätsstandards, zum Teil automatisiert, durchzuführen. Der erfindungsgemäße Expositionsapparat ist daher mit der üblichen Standardausrüstung eines Labors kompatibel und lässt sich zeit- und kostensparend in die etablierten Arbeitsabläufe integrieren. Insbesondere durch die Ausbildung der abgeschlossenen Expositionsatmosphäre innerhalb der bestimmten Wells stellt der erfindungsgemäße Expositionsapparat eine hohe Reproduzierbarkeit und Effektivität der Funktionalität sicher, die insbesondere eine individuelle Beaufschlagung der biologischen Testsysteme ermöglichen. Zudem wird hierdurch ein Vermischen von Fluidphasen, insbesondere von Erhaltungsmedium und Expositionsmedium, sicher vermieden.

Standard-Multiwellplatten sind im Bereich der Zell- und Gewebekulturtechnik als Standardprobengefäße bekannt. Sie dienen zur Aufnahme und zur Untersuchung unterschiedlichster biologischer Testsysteme.

Bei den Standard-Multiwellplatten handelt es sich um laborgängige Einweg-Massenprodukte, die einen hohen Durchsatz von biologischen Testsystemen ermöglichen.

Typischerweise sind solche Standard-Multiwellplatten rechteckig ausgebildet und aus Kunststoff, beispielsweise Polystyren, Polypropylen, Polycarbonat oder Polyethylen, gefertigt.

Dabei sind einzelne Wells, die auch als Vertiefungen oder Kavitäten bezeichnet werden, zur Aufnahme der biologischen Testsysteme auf einer solchen Platte, im Wesentlichen matrixförmig angeordnet. Bei den Wells kann es sich um Aufnahmen handeln, die bereits in der Standard-Multiwellplatte integriert sind, oder um solche, die als separate Kulturgefäße, so genannte Inserts, in vorhandene integrierte Wells oder in entsprechend angeordnete Aussparungen der Standard-Multiwellplatte aufgenommen sind.

Für den erfindungsgemäßen Expositionsapparat sind Multiwellplatten der Konfigurationsgrößen 6-Well, 12-Well, 24-Well, 48-Well oder 96-Well bevorzugt geeignet.

Geeignete Standard-Multiwellplatten werden von einer Reihe von kommerziellen Herstellen angeboten und vertrieben, beispielsweise von Corning Inc., BD Biosciences, Biochrom AG, Greiner GmbH oder der Nunc GmbH & Co. KG.

Standard-Multiwellplatten werden auch als standardisierte PCR (Polymerase Chain Reaction) -Platten, Mikrotiterplatten bzw. HTS (High-Throughput-Screening) - Mikrotiterplatten bezeichnet.

Eine allgemein anerkannte Normierung der Bemaßung und des Aufbaus einer Standard-Multiwellplatte ist durch die Society for Laboratory Automation and Screening (SLAS), einer der American National Standards Institute (ANSI) nahestehenden Gesellschaft von Wissenschaftlern und Ingenieuren, in den Normen (http://www.slas.org/education/microplate.cfm):
- ANSI/SLAS 1-2004: Microplates - Footprint Dimensions
- ANSI/SLAS 2-2004: Microplates - Height Dimensions
- ANSI/SLAS 3-2004: Microplates - Bottom Outside Flange Dimensions
- ANSI/SLAS 4-2004: Microplates - Well Positions
- ANSI/SLAS 6-2012: Microplates - Well Bottom Elevation veröffentlicht.

Es kann von Vorteil sein, wenn zum dichten Abschließen der Expositionsatmosphäre mehr als ein Dichtmittel zwischen Expositionsaufsatz und Standard-Multiwellplatte vorgesehen ist.

Es kann vorteilhaft sein, wenn das Dichtmittel eine das jeweilige Well oder die Öffnung des jeweiligen Wells umlaufende Dichtung ist.

Es kann vorteilhaft sein, wenn das Dichtmittel eine auf die Standard-Multiwellplatte aufbringbare oder aufgebrachte und/oder an der zur Standard-Multiwellplatte weisenden Seite des Expositionsaufsatzes anbringbare oder angebrachte Dichtmatte mit entsprechenden Aussparungen für die bestimmten Wells oder Öffnungen der bestimmten Wells und/oder mit entsprechenden Aussparungen für die den bestimmten Wells zugeordneten Leitungselementen ist.

Erfindungsgemäß ist vorgesehen, dass das jeweils einem bestimmten Well der Standard-Multiwellplatte zugeordnete Leitungselement aus der zur Standard-Multiwellplatte weisenden Seite des Expositionsaufsatz hervorragt. Das Leitungselement kann vorteilhaft rohrfömig ausgebildet sein, insbesondere eine im Wesentlichen rohrförmige Zuleitung für das Expositionsmedium und eine im Wesentlichen rohrförmige Ableitung für das Expositionsmedium aufweisen. Um insbesondere eine gleichmäßige und effektive Exposition des in einem bestimmten Well enthaltenen biologischen Testsystems zu erreichen, kann es vorteilhaft sein, wenn die Zuleitung näher an das biologische Testsystems heranführbar ist als die Ableitung, die Zuleitung also weiter aus der zur Standard-Multiwellplatte weisenden Seite des Expositionsaufsatz hervorragt als die Ableitung. Es kann insbesondere von Vorteil sein, wenn das Leitungselement in platzsparender Weise derart ausgebildet ist, dass die Zuleitung zumindest teilweise innerhalb der Ableitung angeordnet ist und aus dieser in Richtung Well vorsteht. Zuleitung und Ableitung können insoweit zumindest teilweise konzentrisch ausgebildet sein. Vorteilhaft kann die Strömungsführung somit nach dem Rohr-in-Rohr-Prinzip zumindest teilweise entsprechend koaxial im Gegenstrom erfolgen. Es kann vorteilhaft sein, wenn sich die Zuleitung in ihrem zum biologischen Testsystem weisenden Endbereich aufweitet, insbesondere konisch aufweitet.

Erfindungsgemäß ist vorgesehen, dass das Leitungselement nach dem Aufsetzen des Expositionsaufsatzes auf die Standard-Multiwellplatte derart in das Well einbringbar ist, dass ein auf der Außenseite des Leitungselements vorgesehenes und dieses umlaufende Dichtmittel das Leitungselement gegenüber der Innenwand des Wells zur Ausbildung der abgeschlossenen Expositionsatmosphäre dicht abschließt. Das umlaufende Dichtmittel liegt somit nicht auf den Rändern des Wells auf. Wenn das Leitungselement in platzsparender Weise derart ausgebildet ist, dass die Zuleitung jedenfalls teilweise innerhalb der Ableitung angeordnet ist und aus dieser in Richtung Well vorsteht, ist das umlaufende Dichtmittel ein auf der Außenseite der Ableitung vorgesehenes und diese umlaufendes Dichtmittel. Da die Wells üblicherweise einen runden Querschnitt aufweisen, kann es von Vorteil sein, wenn das Dichtmittel ebenfalls einen kreisförmigen Umfang aufweist. Zudem kann es beim Einbringen und Herausziehen des Leitungselements aus dem Well von Vorteil sein, wenn das Dichtmittel zumindest in seinem zur Innenwand des Wells weisenden Endbereich flexibel ausgebildet ist. Dies kann durch eine entsprechende Materialauswahl und/oder dadurch erfolgen, dass das Dichtmittel zur Innenwand des Wells hin dünner wird, die Materialdicke des Dichtmittels ausgehend vom Leitungselement in Richtung Innenwand des Wells also abnimmt. Dadurch kann das Dichtmittel im gewissen Umfang auch universell für unterschiedlich dimensionierte Wells ausgelegt sein.

Es kann vorteilhaft sein, wenn eine dem Multiwellplattenstandard kompatible Multiwellplatte an Stelle der Standard-Multiwellplatte einsetzbar ist. Eine solche in ihrem Format sowie in der Anordnung und Anzahl ihrer Wells kompatible Platte ist beispielsweise in der DE 10 2007 030 413 A1 offenbart, deren Offenbarungsgehalt hiermit durch Bezugnahme ausdrücklich in den Offenbarungsgehalt der vorliegenden Patentanmeldung aufgenommen wird.

Es kann zweckmäßig sein, wenn die Verbindungselemente derart sind, dass der Expositionsaufsatz auf der Standard-Multiwellplatte reversibel festklemmbar ist.

Es kann vorteilhaft sein, wenn die Verbindungselemente Verriegelungselemente umfassen, welche den Expositionsaufsatz und die Standard-Multiwellplatte vorzugsweise unter Spannung zusammenhalten.

Es kann zweckmäßig sein, wenn die Verbindungselemente derart sind, dass die Standard-Multiwellplatte und vorzugsweise die bestimmten Wells während der Durchführung des Experiments festgelegt sind.

Es kann vorteilhaft sein, wenn es sich bei den Verbindungselementen um am Expositionsaufsatz angeordnete Klemmbacken handelt.

Erfindungsgemäß ist vorgesehen, dass der Expositionsaufsatz einen auf die Standard-Multiwellplatte aufsetzbaren und mit dieser mittels der Verbindungselemente verbindbaren Aufsatzhalterahmen aufweist.

Es kann zweckmäßig sein, wenn der Aufsatzhalterahmen die Standard-Multiwellplatte zumindest teilweise umhüllt.

Erfindungsgemäß ist vorgesehen, dass der Expositionsaufsatz einen bewegbaren, mit den Leitungselementen zusammenwirkenden Aufsatzkopf aufweist, wobei das jeweils einem bestimmten Well zugeordnete Leitungselement nach dem Aufsetzen des Expositionsaufsatzes auf die Standard-Multiwellplatte durch Bewegen des Aufsatzkopfes in Richtung Standard-Multiwellplatte in das bestimmte Well bewegbar ist.

Erfindungsgemäß ist vorgesehen, dass der Aufsatzkopf auf dem Aufsatzhalterahmen angeordnet und mit diesem verbunden ist, wobei durch Bewegen des Aufsatzkopfes in Richtung Aufsatzhalterahmen der Aufsatzkopf mittels vorzugsweise am Aufsatzkopf angeordneter Ver- und Entriegelungselemente mit dem Aufsatzhalterahmen zur bzw. während der Durchführung des Experiments derart verriegelbar ist, dass die Leitungselemente festgelegt sind, wobei die Verriegelungselemente den Aufsatzkopf und den Aufsatzhalterahmen vorzugsweise unter Spannung zusammenhalten.

Erfindungsgemäß ist vorgesehen, dass nach der Durchführung des Experiments der Aufsatzkopf mittels der Ver- und Entriegelungselemente von dem Aufsatzhalterahmen entriegelbar ist, derart, dass die Leitungselemente mit Bewegen des Aufsatzkopfes in entgegengesetzter Richtung zur vorherigen aus den Wells lösbar sind. Wenn es sich bei den Wells um Aufnahmen handelt, die als separate Kulturgefäße, so genannte Inserts, in vorhandene integrierte Wells oder in entsprechend angeordnete Aussparungen der Standard-Multiwellplatte aufgenommen bzw. eingehängt sind, hält der Aufsatzhalterahmen das Insert bei entsprechender Entriegelungsbewegung des Aufsatzkopfes zurück bzw. nieder. Dadurch sind die Leitungselemente mit Bewegen des Aufsatzkopfes überhaupt erst aus den Kulturgefäßen bzw. Inserts lösbar. Andernfalls würden sich die Inserts mit der Entriegelungsbewegung des Aufsatzkopfes nachteilig aus der Multiwellplatte heben. Das Halten kann dabei unmittelbar oder mittelbar, beispielsweise über ein im oder am Aufsatzhalterahmen angeordnetes Dichtmittel, erfolgen.

Es kann vorteilhaft sein, wenn zur Durchführung des Experiments zunächst der Aufsatzhalterahmen mit der Standard-Multiwellplatte und anschließend der Aufsatzkopf mit dem Aufsatzhalterahmen verriegelbar ist und dass nach der Durchführung des Experiments zunächst der Aufsatzkopf vom Aufsatzhalterahmen entriegelbar und anschließend der Aufsatzhalterahmen von der die Standard-Multiwellplatte entriegelbar ist.

Es kann zweckmäßig sein, wenn der Expositionsapparat eine Basisaufnahme zur Aufnahme der Standard-Multiwellplatte aufweist.

Es kann von Vorteil sein, wenn die Basisaufnahme die Standard-Multiwellplatte zumindest teilweise umhüllt.

Es kann vorteilhaft sein, wenn der Expositionsaufsatz, insbesondere der Aufsatzhalterahmen des Expositionsaufsatzes, mittels wenigstens einer Positionierhilfe auf der Basisaufnahme positionierbar und aufsetzbar ist.

Es kann vorteilhaft sein, wenn die Positionierhilfe einen auf der Basisaufnahme angeordneten Stift und eine für diesen entsprechend vorgesehene Aussparung im Expositionsaufsatz, insbesondere im Aufsatzhalterahmen des Expositionsaufsatzes, aufweist.

Es kann vorteilhaft sein, wenn die in der Basisaufnahme aufgenommene Standard-Multiwellplatte von der Basisaufnahme und dem auf der Standard-Multiwellplatte und der Basisaufnahme aufgesetzten Expositionsaufsatz bzw. Aufsatzhalterahmen des Expositionsaufsatzes nach dem Verriegeln vollständig umhüllt ist.

Es kann vorteilhaft sein, wenn das durch die Leitungselemente leitbare Expositionsmedium flüssig oder gasförmig ist, wobei das Expositionsmedium mit oder ohne Testsubstanz versehbar ist.

Es kann vorteilhaft sein, wenn zwischen Aufsatzhalterahmen und Aufsatzkopf ein das Leitungselement umlaufendes Dichtmittel vorgesehen ist.

Es kann vorteilhaft sein, wenn der Expositionsaufsatz, der Aufsatzkopf, der Aufsatzhalterahmen und/oder die Basisaufnahme ganz oder teilweise, jeweils für sich oder zumindest teilweise zusammen temperierbar, insbesondere elektrisch beheizbar sind, vorzugsweise derart, dass sich innerhalb des Expositionsapparats ein thermischer Gradient ausbildet, der insbesondere zur gezielten Abscheidung von Substanzen aus einem Aerosolstrom mittels Thermophorese nutzbar ist. Für die Ausbildung eines Temperaturgradienten ist insbesondere der Einsatz von Peltier-Elementen vorteilhaft.

Durch die gezielte Temperierung oben genannter Teile bzw. Bauteile liegt im Aerosolstrom ein Temperaturgradient vor, wodurch auf die im Aerosolstrom enthaltenen Partikel eine Kraft in Richtung der niedrigeren Temperatur wirkt. So können Partikel gezielt dem biologischen Testsystem zugeführt werden. Es kann zur Einstellung eines Temperaturgradienten vorteilhaft sein, wenn der Expositionsaufsatz, der Aufsatzkopf, der Aufsatzhalterahmen und/oder die Basisaufnahme ganz oder teilweise, vorzugsweise durch Isoliermittel, gegeneinander isoliert sind. Vorzugsweise können hierzu vorhandene Dichtmittel auch als Isoliermittel ausgebildet sein.

Es kann von Vorteil sein, wenn der Boden wenigstens eines Wells der Standard-Multiwellplatte als permeabler Träger ausgebildet ist oder einen solchen gegebenenfalls auch zusätzlich aufweist, wobei auf diesem das biologische Testsystem angeordnet ist.

Vorzugsweise ist der permeable Träger derart ausgebildet, dass er einerseits zur Aufnahme des biologischen Testsystems geeignet ist und andererseits eine physikalische Trennung der die Expositionsatmosphäre bildenden flüssigen oder gasförmigen Phase von der das Erhaltungsmedium bildenden flüssigen Phase ermöglicht. Mithin kann die Porengröße und Porendichte des permeablen Trägers in Abhängigkeit vom der Art des biologischen Prüfsystems und der Art sowie Viskosität des Erhaltungsmediums variieren.

Vorzugsweise kann der permeable Träger eine mikroporöse Membran sein. Zur Aufnahme des biologischen Testsystems kann das Well auch durch ein in die Multiwellplatte einbringbares kommerziell erhältliches so genanntes Cell Culture Insert ausgeführt sein, wobei der permeable Träger die Bodenmembran des Wells bildet.

Es kann vorteilhaft sein, wenn wenigstens ein Zu- und Ableitungssystem vorgesehen ist, dass mit einem oder mehr als einem Leitungselement aus Zu- und Ableitung zur perfusiven Exposition der technischen Replikate wenigstens eines biologischen Testsystems verbunden oder verbindbar ist.

Das Zuleitungssystem umfasst alle Vorrichtungen, die geeignet sind, das flüssige oder gasförmige Expositionsmedium über die Oberfläche des biologischen Testsystems zur Bildung einer Expositionsatmosphäre oberhalb des biologischen Testsystems zu leiten.

Das Zuleitungssystem kann auch alle Vorrichtungen umfassen, die durch ihre Konstruktion die Heranführung des flüssigen oder gasförmigen Expositionsmediums in besonderer Art realisieren, beispielsweise mit hyperbolischem Innenprofil zur gezielten Abscheidung von Aerosoltröpfchen.

Ferner kann das Zuleitungssystem vorzugsweise auch Vorrichtungen zur elektrostatischen Abscheidung von Partikeln oder Tröpfchen und/oder eine Aufladungsvorrichtung. Derartige Vorrichtungen sind beispielsweise aus der DE 195 26 533 A1 bekannt. Die Offenbarung dieser Patentanmeldung wird hiermit ebenfalls durch Bezugnahme vollinhaltlich in die vorliegende Anmeldung mit aufgenommen.

Es kann zweckmäßig sein, wenn eine Aufteilung des Expositionsmediums auf die technischen Replikate bzw. auf die bestimmten Wells mittels der diesen Wells zugeordneten Leitungselemente vorgesehen ist, wobei die Aufteilung insbesondere linear, radial, horizontal und/oder vertikal ausführbar ist.

Es kann vorteilhaft sein, wenn die Aufteilung aus unterschiedlichen Expositionsmedien ausführbar ist.

Es kann vorteilhaft sein, wenn die Zuleitung bzw. das Zuleitungssystem mit zumindest einer das Expositionsmedium aufbereitenden Einrichtung, vorzugsweise einer solchen zur Thermostatisierung des Expositionsmediums, und/oder einer das Expositionsmedium detektierenden Einrichtung in Verbindung steht.

Es kann zweckmäßig sein, wenn das die Zu- und/oder Ableitung und/oder Zu- und/oder Ableitungssystem des jeweiligen Leitungselements derart ausgebildet sind, dass das Expositionsmedium kontrolliert vorzugsweise mittels eines an der Ableitung bzw. am Ableitungssystem anliegenden Unterdrucks durch das Well bzw. über das biologische Testsystems geleitet bzw. gefördert wird.

Es kann vorteilhaft sein, wenn die Ableitung bzw. das Ableitungssystem mit wenigstens einem Anschluss für eine einen Unterdruck erzeugende Vorrichtung, insbesondere eine Pumpe, beispielsweise eine Peristaltik-Pumpe für flüssige oder eine Membranpumpe für gasförmige Medien, versehen ist.

Es kann vorteilhaft sein, wenn die Zu- und/oder Ableitung bzw. das Zu- und/oder Ableitungssystem des Expositionsapparats, insbesondere des Expositionsaufsatzes, mit Steuerelementen zur gleichmäßigen Verteilung des Expositionsmediums auf bestimmte Wells verbindbar oder verbunden sind oder dieselben enthalten. Bei den Steuerelementen kann es sich beispielsweise um Massenflusscontroller sowie konstruktive Vorrichtungen zur Strömungsmodulierung und/oder Ventile handeln.

Es kann vorteilhaft sein, wenn der Expositionsapparat ein oder mehr als ein Modul aufweist bzw. mit diesem verbindbar ist, derart, dass das oder die zu- und/oder abgeleiteten Expositionsmedien einer physikalisch/chemischen oder biologischen Aufbereitung unterziehbar sind. Hierbei kann es sich beispielsweise um eine Befeuchtung, eine CO₂-Anreicherung, eine elektrostatische Aufladung oder Neutralisierung, ein Spiken mit Referenzpartikeln oder Gasen, ein Einspeisen von biologisch aktive Komponenten wie Antikörpern, Pollen, etc. und/oder um eine Thermostatisierung handeln.

Es kann vorteilhaft sein, wenn die Aufbereitung des Expositionsmediums für alle technischen Replikate bzw. alle bestimmten Wells gleichartig oder für einzelne der bestimmten Wells unterschiedlich ausführbar ist.

Es kann zweckmäßig sein, wenn in einem Modul mehrere Aufbereitungsverfahren in Kombination oder nur einzeln anwendbar sind.

Es kann vorteilhaft sein, wenn das wenigstens eine Modul zur Aufbereitung vor und/oder nach wenigstens einem Modul zur Detektion des oder der zu- und/oder abgeleiteten Expositionsmedien angeordnet ist.

Es kann vorteilhaft sein, wenn der Expositionsapparat ein oder mehr als ein Modul aufweist bzw. mit diesem verbindbar ist, derart, dass das oder die zu- und/oder abgeleiteten Expositionsmedien und/oder das biologische Testsystem einer physikalisch/chemischen oder biologischen Analyse unterziehbar ist. Bei den Analyseverfahren kann es sich beispielsweise um eine Streulichtmessung, pH-Wert-Bestimmung, um spektroskopische Analysemethoden oder um Antikörper-basierte Analyseverfahren handeln.

Es kann zweckmäßig sein, wenn die Analyse des Expositionsmediums für alle technischen Replikate bzw. alle bestimmten Wells gleichartig oder für einzelne der technischen Replikate bzw. er bestimmten Wells unterschiedlich ausführbar ist.

Es kann zweckmäßig sein, wenn in einem Modul mehrere Analyseverfahren in Kombination oder nur einzeln anwendbar sind.

Es kann vorteilhaft sein, wenn das wenigstens eine Modul zur Analyse vor und/oder nach wenigstens einem Modul zur Aufbereitung des oder der zu- und/oder abgeleiteten Expositionsmedien angeordnet ist.

Es kann vorteilhaft sein, wenn ein Modul zur physikalisch/chemischen oder biologischen Analyse innerhalb oder unterhalb der Standard-Multiwellplatte ausführbar ist

Es kann zweckmäßig sein, wenn wenigstens ein Well, eine Gruppe von Wells oder jedes Well einer Gruppe derart über das jeweils einem Well zugeordnete Leitungselement aus Zu- und Ableitung mit einem Zu- und Ableitungssystem in Verbindung steht, das jedes Well, jede Gruppe von Wells oder jedes Well einer Gruppe individuell mit gleichen oder unterschiedlichen Expositionsvolumenströmen eines Expositionsmediums mit oder ohne Testsubstanz durchströmt wird.

Es kann vorteilhaft sein, wenn neben dem jeweils über das Leitungselement in das Well und aus dem Well geleiteten Expositionsvolumenstrom mindestens ein weiterer Volumenstrom vorgesehen ist, über den das Expositionsmedium nach Art eine Vorförderung durch den Expositionsapparat führbar ist, wobei vorgebbare Expositionsvolumenströme aus diesem weiteren Volumenstrom leitbar bzw. speisbar sind.

Es kann vorteilhaft sein, wenn der mindestens eine weitere Volumenstrom nach Art einer Vorförderung mit einer anderen Volumenflussrate, vorzugsweise größeren Volumenflussrate, betreibbar ist als die Summe der einzelnen Expositionsvolumenströme.

Es kann vorteilhaft sein, wenn mehrere weitere Volumenströme vorgesehen sind, wobei mit jedem der weiteren Volumenströme vorgebbare Expositionsvolumenströme in Verbindung bringbar sind.

Es kann zweckmäßig sein, wenn einem oder mehr als einem Well der Standard-Multiwellplatte kein Leitungselement zugeordnet ist, wobei das Well oder diese Gruppe von Wells als so genannte Kontrollprobe im in-vitro-Experiment mitführbar ist.

Es kann vorteilhaft sein, wenn einem oder mehr als einem Well je ein Leitungselement zugeordnet ist, durch das das Expositionsmedium ohne Testsubstanz dem Well oder dieser Gruppe von Wells zuleitbar und als so genannte Blindprobe im in-vitro-Experiment mitführbar ist.

Es kann vorteilhaft sein, wenn einem oder mehr als einem Well je ein Leitungselement zugeordnet ist, durch das das Expositionsmedium mit Testsubstanz dem Well oder dieser Gruppe von Wells zuleitbar und als eigentliche Probe im in-vitro-Experiment mitführbar ist.

Es kann vorteilhaft sein, wenn der Standard-Multiwellplatte ein Erhaltungsmedium zuführbar oder zugeordnet ist, das zur Versorgung des biologischen Testsystems in direktem Kontakt mit der Unterseite des Wells bzw. des permeablen Trägers steht, auf der bzw. dem das biologische Testsystem angeordnet ist.

Es kann zweckmäßig sein, wenn der Expositionsapparat ein oder mehr als ein Modul aufweist bzw. mit diesem verbindbar ist, um das Erhaltungsmedium oder um das oder die zu- und/oder abgeführten Erhaltungsmedien einer physikalisch/chemischen oder biologischen Aufbereitung zu unterziehen. Bei der Aufbereitung kann es sich beispielsweise um den Zusatz biologisch aktiver Substanzen, um den Zusatz von metaboliserenden Proteinen, um den Zusatz von Säure/Base zur pH-Wert-Regulierung oder um eine Thermostatisierung handeln.

Es kann zweckmäßig sein, wenn die Aufbereitung des Erhaltungsmediums für alle technischen Replikate bzw. für alle bestimmten Wells gleichartig oder für einzelne der technischen Replikate bzw. der bestimmten Wells unterschiedlich ausführbar ist.

Es kann vorteilhaft sein, wenn in einem Modul mehrere Aufbereitungsverfahren in Kombination oder nur einzeln anwendbar sind.

Es kann zweckmäßig sein, wenn das wenigstens eine Modul zur Aufbereitung vor und/oder nach wenigstens einem Modul zur Detektion des Erhaltungsmediums oder des oder der zu- und/oder abgeführten Erhaltungsmedien angeordnet ist.

Es kann vorteilhaft sein, wenn der Expositionsapparat ein oder mehr als ein Modul aufweist bzw. mit diesem verbindbar ist, derart, dass das Erhaltungsmedium oder das oder die zu- und/oder abgeführten Erhaltungsmedien einer physikalisch/chemischen oder biologischen Analyse unterziehbar sind. Dabei kann es sich beispielhaft um eine Streulichtmessung, eine pH-Wert-Bestimmung, um spektroskopische Analysemethoden oder um Antikörper-basierte Analyseverfahren handeln.

Es kann vorteilhaft sein, wenn die Analyse des Erhaltungsmediums für alle technischen Replikate bzw. für alle bestimmten Wells gleichartig oder für einzelne der technischen Replikate bzw. bestimmten Wells unterschiedlich ausführbar ist.

Es kann vorteilhaft sein, wenn das wenigstens eine Modul zur Analyse vor und/oder nach wenigstens einem Modul zur Aufbereitung des Erhaltungsmedium oder des oder der zu- und/oder abgeführten Expositionsmedien angeordnet ist.

Es kann vorteilhaft sein, wenn ein Modul zur physikalisch/chemischen oder biologischen Analyse innerhalb oder unterhalb der Standard-Multiwellplatte ausführbar ist.

Nachfolgend wird die Erfindung anhand von Ausführungsbeispielen erläutert, die in der Zeichnung dargestellt sind. In dieser zeigen
- Fig. 1: schematisch im Querschnitt einen erfindungsgemäßen Expositionsapparat mit einer Multiwellplatte in geöffneter Stellung,
- Fig. 2: schematisch im Querschnitt den erfindungsgemäßen Expositionsapparat aus Fig. 1 mit Multiwellplatte in einer ersten Verriegelungsstellung,
- Fig. 3: schematisch im Querschnitt den erfindungsgemäßen Expositionsapparat aus Fig. 1 mit Multiwellplatte in einer zweiten Verriegelungsstellung,
- Fig. 4: eine schematische Darstellung zur Funktion des erfindungsgemäßen Expositionsapparats während eines Experiments,
- Fig. 5: ein erstes Anwendungs- bzw. Ausführungsbeispiel des erfindungsgemäßen Expositionsapparats,
- Fig. 6: ein zweites Anwendungs- bzw. Ausführungsbeispiel des erfindungsgemäßen Expositionsapparats,
- Fig. 7: ein drittes Anwendungs- bzw. Ausführungsbeispiel des erfindungsgemäßen Expositionsapparats,
- Fig. 8: ein viertes Anwendungs- bzw. Ausführungsbeispiel des erfindungsgemäßen Expositionsapparats,
- Fig. 9: ein fünftes Anwendungs- bzw. Ausführungsbeispiel des erfindungsgemäßen Expositionsapparats und
- Fig. 10: schematisch eine mögliche Aufteilung des Expositionsmediums auf technische Replikate, die jeweils biologischen Testsystemen zugeordnet sind.

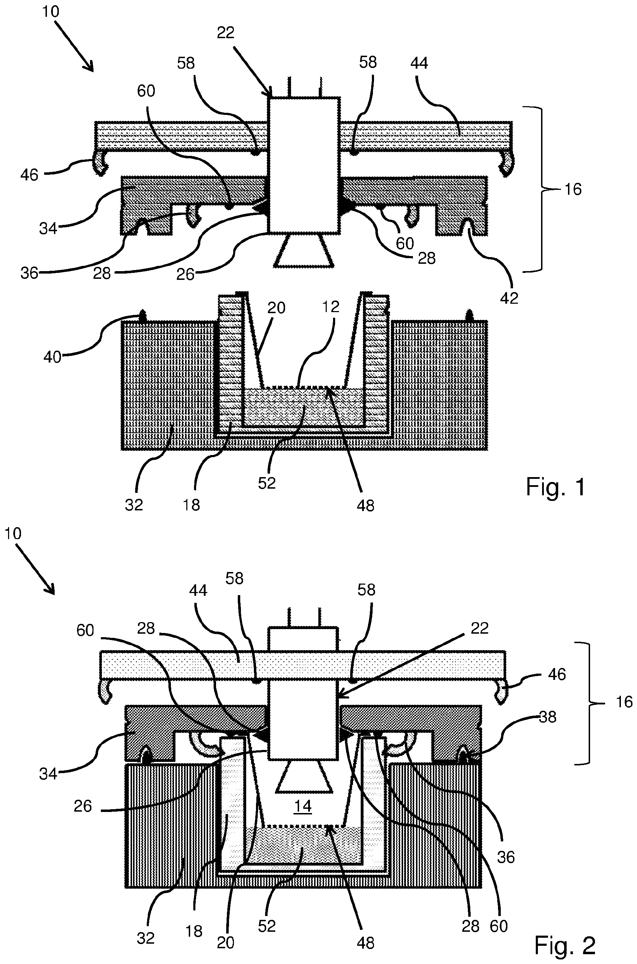

Werden in den Figuren 1 bis 11 gleiche Bezugsziffern verwendet, so bezeichnen diese gleiche Teile oder Bauteile, so dass zwecks Vermeidung von Wiederholungen nicht bei jeder Figurenbeschreibung erneut auf bereits beschriebene Teile bzw. Bauteile eingegangen werden muss.

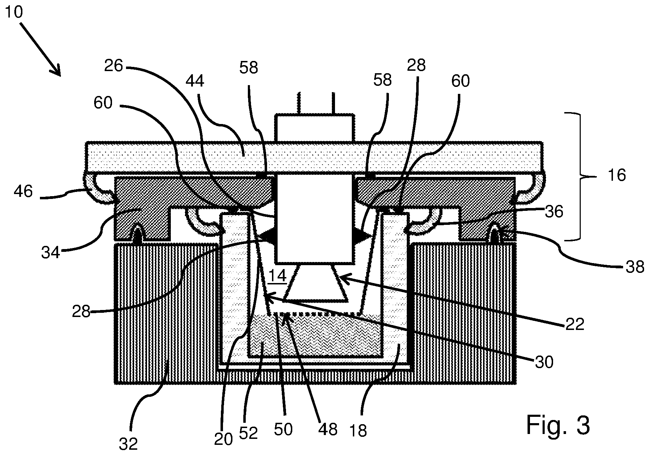

Fig. 1 bis 3 zeigen schematisch im Querschnitt einen erfindungsgemäßen Expositionsapparat 10 mit einer Multiwellplatte 18 in geöffneter Stellung (Fig.1), in einer ersten Verriegelungsstellung (Fig. 2) und in einer zweiten Verriegelungsstellung (Fig. 3). Bei der Multiwellplatte 18 handelt es sich um eine Standard-Multiwellplatte, wobei übersichtshalber nur ein Well 20 der Standard-Multiwellplatte 18 dargestellt ist.

Der gezeigte Expositionsapparat 10 dient zur Durchführung von in-vitro-Experimenten mit technischen Replikaten wenigstens eines biologischen Testsystems 12. Die technischen Replikate mit ihren entsprechenden biologischen Testsystemen 12 werden hierbei perfusiv unter Ausbildung einer Expositionsatmosphäre 14 mit einem Expositionsmedium 24 beaufschlagt.

Der Expositionsaufsatz 16 ist derart dimensioniert, dass dieser auf die Standard-Multiwellplatte 18 aufsetzbar und mit dieser 18 mittels geeigneter Verbindungselemente, vorliegend mittels Verriegelungselementen 36, beispielsweise in Form von Klemmbacken, fest verbindbar und verriegelbar ist.

Der Expositionsaufsatz 16 weist mehrere Leitungselemente 22 mit jeweils zumindest einer Zuleitung 54 und zumindest einer Ableitung 56 für ein Expositionsmedium 24 auf, wobei jedes Leitungselement 22 einem bestimmten, vorzugsweise mit biologischem Testsystem 12 versehenen Well 20 der Standard-Multiwellplatte 18 zur Ausbildung der Expositionsatmosphäre 14 innerhalb des bestimmten Wells 20 zugeordnet ist.

Der Expositionsaufsatz 16 ist derart auf die Standard-Multiwellplatte 18 aufsetzbar und mit dieser verbind- bzw. verriegelbar, dass die sich während des in-vitro-Experiments innerhalb des bestimmten Wells 20 ausbildendende Expositionsatmosphäre 14 dicht abgeschlossen ist. Expositionsmedium 14 kann unterdessen nur über das jeweils zugeordnete Leitungselement 22 dem bestimmten 20 zugeleitet und aus diesem abgeleitet werden.

Zum dichten Abschließen der Expositionsatmosphäre 14 ist ein oder mehr als ein Dichtmittel 28, 60 zwischen Expositionsaufsatz 16 und Standard-Multiwellplatte 18 vorgesehen.

Das Dichtmittel 28, 60 ist eine das jeweilige Well 20 bzw. die Öffnung des jeweiligen Wells 20 und/oder eine das jeweilige Leitungselement 22 umlaufende Dichtung ist.

Das Dichtmittel 60 ist eine an der zur Standard-Multiwellplatte 18 weisenden Seite des Expositionsaufsatzes 16 angebrachte Dichtmatte mit entsprechenden Aussparungen für die bestimmten Wells 20 bzw. Öffnungen der bestimmten Wells 20 und/oder mit entsprechenden Aussparungen für die den bestimmten Wells 20 zugeordneten Leitungselementen 22 ist. Ein solches Dichtmittel 60 kann zur Ausbildung einer abgeschlossenes Expositionsatmosphäre 14 ausreichend sein, wenn die das bestimmte Well 20 keine an der Oberkannte des Wells vorhandene seitlichen Öffnungen, die eine Verbindung zum Erhaltungsmedium offen lassen, aufweist. Diese Dichtmittel 60 können ausreichend sein, wenn das Well 20 über seinen Umfang durchgehend an die Grundplatte der Standard-Multiwellplatte 18 anschließt. Üblicherweise ist ein solches Dichtmittel nicht ausreichend, wenn das Well 20 - wie vorliegend - als ein so genanntes Insert in die Standard-Multiwellplatte 18 einhängbar ist.

Zu diesem Zweck ist vorgesehen, dass das jeweils einem bestimmten Well 20 der Standard-Multiwellplatte 18 zugeordnete Leitungselement 22 aus der zur Standard-Multiwellplatte 18 weisenden Seite des Expositionsaufsatzes 16 hervorragt.

Das Leitungselement 22 wird hierbei mit dem und/oder nach Aufsetzen des Expositionsaufsatzes 16 auf die Standard-Multiwellplatte 18 derart in das Well 20 eingebracht, dass ein auf der Außenseite 26 des Leitungselements 22 vorgesehenes und dieses umlaufende Dichtmittel 28 das Leitungselement 22 gegenüber der Innenwand 30 des Wells 20 zur Ausbildung der abgeschlossenen Expositionsatmosphäre 14 dicht abschließt.

Der vorliegende Expositionsaufsatz 16 weist einen auf die Standard-Multiwellplatte 18 aufsetzbaren und mit dieser mittels der Verbindungs- bzw. Verriegelungselemente 36 verbindbaren Aufsatzhalterahmen 34 auf. Nach dem Aufsetzen umhüllt der Aufsatzhalterahmen 34 die Standard-Multiwellplatte 18, wie in Fig. 3 gut zu erkennen, teilweise.

Der Expositionsaufsatz 16 weist außerdem einen bewegbaren, mit den Leitungselementen 22 zusammenwirkenden Aufsatzkopf 44 auf, wobei das jeweils einem bestimmten Well 20 zugeordnete Leitungselement 22 nach dem Aufsetzen des Expositionsaufsatzes 16 auf die Standard-Multiwellplatte 18 durch Bewegen des Aufsatzkopfes 44 in Richtung Standard-Multiwellplatte 18 in das bestimmte Well 20 bewegbar ist. Der Aufsatzkopf 44 ist hierbei auf dem Aufsatzhalterahmen 34 angeordnet und mit diesem verbunden, wobei durch Bewegen des Aufsatzkopfes 44 in Richtung Aufsatzhalterahmen 34 der Aufsatzkopf 14 mittels vorzugsweise am Aufsatzkopf 44 angeordneter Ver- und Entriegelungselemente 46 mit dem Aufsatzhalterahmen 34 zur bzw. während der Durchführung des Experiments derart verriegelbar ist, dass die Leitungselemente 22 festgelegt sind, wobei die Verriegelungselemente 46 den Aufsatzkopf 44 und den Aufsatzhalterahmen 34 vorzugsweise unter Spannung zusammenhalten.

Nach der Durchführung des Experiments ist der Aufsatzkopf 44 mittels der Ver- und Entriegelungselemente 46 auch wieder von dem Aufsatzhalterahmen 34 entriegelbar, derart, dass die Leitungselemente 22 mit Bewegen des Aufsatzkopfes 44 in entgegengesetzter Richtung zur vorherigen aus den Wells 20 lösbar sind, was vorliegend nicht dargestellt ist. Wenn - wie in den Fig. 1 bis 3 dargestellt - Kulturgefäße bzw. so genannte Inserts 20 in die Kavitäten der Standard-Multiwellplatte 18 eingehängt sind, ermöglicht die vorgenannte zweistufige Entriegelung ein sicheres Entfernen des jeweiligen Leitungselements 22 aus dem Insert 20, da das Insert 20 bei entsprechender Entriegelungsbewegung des Aufsatzkopfes 48 durch den Aufsatzhalterahmen 34 zurück- bzw. niedergehalten wird. Andernfalls würde sich das Insert 20 mit der Entriegelungsbewegung des Aufsatzkopfes 48 nachteilig aus der Multiwellplatte 18 heben. Das Insert 20 wäre dann also nicht mehr zur weiteren Verwendung, beispielsweise Analyse, in der Multiwellplatte 18 positioniert.
Zur Durchführung des Experiments ist also zunächst der Aufsatzhalterahmen 34 mit der Standard-Multiwellplatte 18 und anschließend der Aufsatzkopf 44 mit dem Aufsatzhalterahmen 34 verriegelbar. Nach der Durchführung des Experiments ist zunächst der Aufsatzkopf 44 vom Aufsatzhalterahmen 34 entriegelbar und anschließend der Aufsatzhalterahmen 34 von der die Standard-Multiwellplatte 18 entriegelbar.

Der erfindungsgemäße Expositionsapparat 10 weist quasi als weiteres Modul eine Basisaufnahme 32 zur Aufnahme der Standard-Multiwellplatte 18 auf. Der Aufsatzhalterahmen 34 des Expositionsaufsatzes 16 ist hierbei mittels wenigstens einer Positionierhilfe 38 auf der Basisaufnahme 32 positionier- und aufsetzbar. Die Positionierhilfe 38 weist mehrere auf der Basisaufnahme 32 angeordnete Stifte 40 und jeweils eine für jeden Stift 40 entsprechend vorgesehene Aussparung 42 im Aufsatzhalterahmen 34 des Expositionsaufsatzes 16 auf.

Wie in Fig. 3 gut zu erkennen ist, wird die Standard-Multiwellplatte 18 von der Basisaufnahme 32 teilweise umhüllt, wobei im zusammengesetzten Zustand von Expositionsaufsatz 16 und Basisaufnahme 32 die Standard-Multiwellplatte 18 komplett umhüllt ist.

Zwischen Aufsatzhalterahmen 34 und Aufsatzkopf 44 ist ein weiteres das Leitungselement 22 umlaufendes Dichtmittel 58 vorgesehen.

Vorteilhaft sind der Aufsatzkopf 44, der Aufsatzhalterahmen 34 und die Basisaufnahme 32 temperierbar, insbesondere elektrisch beheizbar, vorzugsweise derart, dass sich innerhalb des - wie in Fig. 3 dargestellt - zusammengesetzten Expositionsapparats 10 ein thermischer Gradient ausbildet, der insbesondere zur gezielten Abscheidung von Testsubstanzen aus einem Aerosolstrom als Expositionsmedium 24 mittels Thermophorese nutzbar ist

In den Fig. 1 bis 3 ist gut zu erkennen, dass der Boden 48 eines Wells 20 der Standard-Multiwellplatte 18 als permeabler Träger 50 ausgebildet ist oder einen solchen 50 aufweist, wobei auf diesem das biologische Testsystem 12 angeordnet bzw. angezüchtet ist.

Der Standard-Multiwellplatte 32 kann ein Erhaltungsmedium 52 zugeordnet sein, das zur Versorgung des biologischen Testsystems 12 in direktem Kontakt mit der Unterseite des Wells 20 bzw. des permeablen Trägers 50 steht, auf der bzw. dem das biologische Testsystem angeordnet ist.

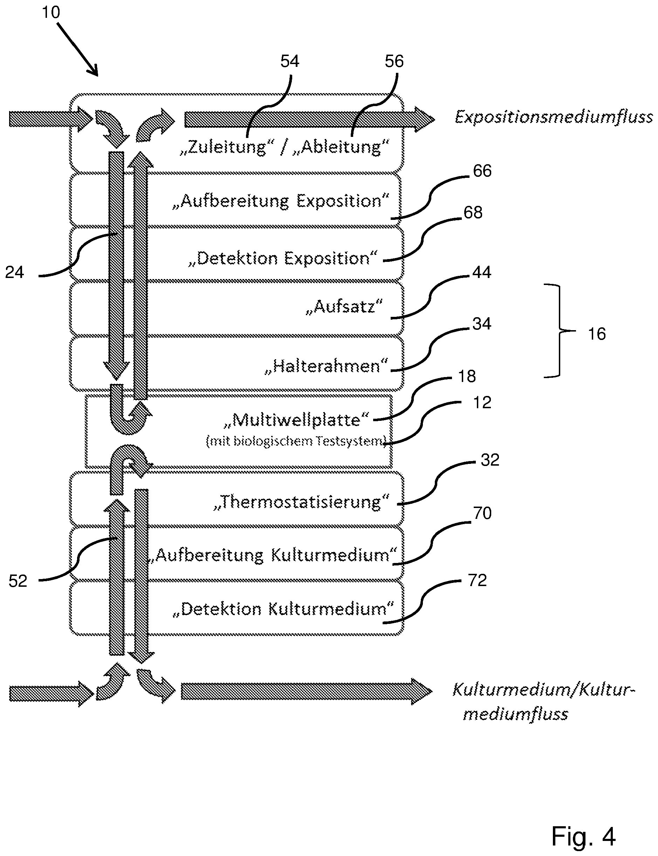

Fig. 4 zeigt eine schematische Darstellung zur Funktion des erfindungsgemäßen Expositionsapparats 10 während eines Experiments. Dargestellt sind verschiedene Funktionsmodule, die dem erfindungsgemäßem Expositionsapparat 10 zugeordnet sind. Zudem ist der Fluss des Expositionsmediums 24 und des Erhaltungs- bzw. Kulturmediums 52 jeweils in Verbindung mit den einzelnen Funktionsmodulen dargestellt, wobei das Kulturmedium 52 überwiegend statisch, also in nicht fließender Weise dem jeweiligen biologischen Testsystem zugeordnet ist.

Die Funktionsmodule "Zuleitung"/"Ableitung", "Aufsatz" bzw. Aufsatzkopf 44, "Halterahmen" bzw. Aufsatzhalterahmen 34, "Multiwellplatte" bzw. Standard-Multiwellplatte 18 mit biologischem Testsystem sowie "Thermostatisierung" bzw. temperierbare Basisaufnahme 32 wurden bereits diskutiert.

Vorteilhaft kann die Zuleitung 54 und/oder Ableitung 56 mit zumindest einer das Expositionsmedium 24 aufbereitenden Einrichtung 66 als wenigstens ein weiteres Modul, beispielsweise einer solchen zur Thermostatisierung des Expositionsmediums 24, in Verbindung stehen. Vorteilhaft kann die Zuleitung 54 und/oder Ableitung 56 mit zumindest einer das Expositionsmedium 24 detektierenden Einrichtung 68 als wenigstens ein weiteres Modul in Verbindung stehen.

Die Zu- und/oder Ableitung 54, 56 und/oder Zu- und/oder Ableitungssystem des jeweiligen Leitungselements 22 sind derart ausgebildet, dass das Expositionsmedium 24 kontrolliert vorzugsweise mittels eines an der Ableitung 56 bzw. am Ableitungssystem anliegenden Unterdrucks durch das Well 20 bzw. über das biologische Testsystems geleitet bzw. gefördert wird.

Die Ableitung 56 bzw. das Ableitungssystem ist mit wenigstens einem Anschluss für eine einen Unterdruck erzeugende Vorrichtung, insbesondere eine Peristaltik-Pumpe für flüssige oder eine Membranpumpe für gasförmige Medien, versehen.

Die Zu- und/oder Ableitung 54, 56 bzw. das Zu- und/oder Ableitungssystem des Expositionsapparats 10, insbesondere des Expositionsaufsatzes 16, kann vorteilhaft mit hier nicht dargestellten Steuerelementen zur gleichmäßigen Verteilung des Expositionsmediums 24 auf bestimmte Wells 20 der Standard-Multiwellplatte 18 verbindbar oder verbunden sein. Bei den Steuerelementen kann es sich beispielsweise um Massenflusscontroller sowie konstruktive Vorrichtungen zur Strömungsmodulierung und/oder Ventile handeln.

Der Expositionsapparat 10 weist vorteilhaft ein oder mehr als ein Modul auf bzw. ist mit diesem verbindbar, derart, dass das oder die zu- und/oder abgeleiteten Expositionsmedien einer physikalisch/chemischen oder biologischen Aufbereitung unterziehbar sind. Hierbei kann es sich beispielsweise um eine Befeuchtung, eine CO₂-Anreicherung, eine elektrostatische Aufladung oder Neutralisierung, ein Spiken mit Referenzpartikeln oder Gasen, ein Einspeisen von biologisch aktive Komponenten wie Antikörpern, Pollen, etc. und/oder um eine Thermostatisierung handeln.

Die Aufbereitung 66 des Expositionsmediums 24 kann für alle technischen Replikate bzw. alle bestimmten Wells 20 gleichartig oder für einzelne der technischen Replikate bzw. der bestimmten Wells 20 unterschiedlich ausführbar sein. Dadurch können Test- und Kontrollgruppen gebildet werden.

In einem Modul können mehrere Aufbereitungsverfahren in Kombination oder nur einzeln anwendbar sein.

Das wenigstens eine Modul zur Aufbereitung kann vor und/oder nach wenigstens einem im Folgenden beschriebenen Modul zur Detektion des oder der zu- und/oder abgeleiteten Expositionsmedien angeordnet.

Der erfindungsgemäße Expositionsapparat 10 weist ein oder mehr als ein Modul 68 auf bzw. ist mit diesem verbindbar, derart, dass das oder die zu- und/oder abgeleiteten Expositionsmedien und/oder das biologische Testsystem einer physikalisch/chemischen oder biologischen Analyse unterziehbar sind. Bei den Analyseverfahren kann es sich beispielsweise um eine Streulichtmessung, pH-Wert-Bestimmung, um spektroskopische Analysemethoden oder um Antikörper-basierte Analyseverfahren handeln.

Die Analyse des Expositionsmediums 24 ist für alle technischen Replikate bzw. alle bestimmten Wells 20 gleichartig oder für einzelne der technischen Replikate bzw. der bestimmten Wells 20 unterschiedlich ausführbar.

In einem Modul sind mehrere Analyseverfahren in Kombination oder nur einzeln anwendbar.

Wenigstens ein Modul ist zur Analyse vor und/oder nach wenigstens einem vorangegangen beschriebenen Modul zur Aufbereitung des oder der zu- und/oder abgeleiteten Expositionsmedien angeordnet.

Vorteilhaft kann ein Modul zur physikalisch/chemischen oder biologischen Analyse innerhalb oder unterhalb der Standard-Multiwellplatte 18 ausführbar sein.

Wie bereits ausgeführt ist der Standard-Multiwellplatte (18) ein Erhaltungsmedium 52 zuführbar oder zugeordnet, das zur Versorgung des biologischen Testsystems in direktem Kontakt mit der Unterseite des Wells 20 bzw. des permeablen Trägers 50 steht, auf der bzw. dem das biologische Testsystem angeordnet ist.

Der erfindungsgemäße Expositionsapparat 10 weist vorteilhaft ein oder mehr als ein Modul auf bzw. ist mit diesem verbindbar, um das Erhaltungsmedium 52 oder um das oder die zu- und/oder abgeführten Erhaltungsmedien 52 einer physikalisch/chemischen oder biologischen Aufbereitung zu unterziehen. Bei der Aufbereitung kann es sich beispielsweise um den Zusatz biologisch aktiver Substanzen, um den Zusatz von metaboliserenden Proteinen, um den Zusatz von Säure/Base zur pH-Wert-Regulierung oder um eine Thermostatisierung handeln.

Die Aufbereitung des Erhaltungsmediums 52 kann für alle technischen Replikate bzw. für alle bestimmten Wells 20 gleichartig oder für einzelne der technischen Replikate bzw. der bestimmten Wells 20 unterschiedlich ausführbar sein. So lassen sich Kontroll- und Testgruppen bilden.

In einem Modul sind mehrere Aufbereitungsverfahren in Kombination oder nur einzeln anwendbar.

Wenigstens ein Modul 70 zur Aufbereitung ist vor und/oder nach wenigstens einem im Folgenden beschriebenen Modul 72 zur Detektion des Erhaltungsmediums 52 oder des oder der zu- und/oder abgeführten Erhaltungsmedien 52 angeordnet ist.

Der erfindungsgemäße Expositionsapparat 10 weist ein oder mehr als ein Modul 72 auf bzw. ist mit diesem verbindbar, derart, dass das Erhaltungsmedium 52 oder das oder die zu- und/oder abgeführten Erhaltungsmedien 52 einer physikalisch/chemischen oder biologischen Analyse unterziehbar sind. Dabei kann es sich beispielhaft um eine Streulichtmessung, eine pH-Wert-Bestimmung, um spektroskopische Analysemethoden oder um Antikörper-basierte Analyseverfahren handeln.

Die Analyse des Erhaltungsmediums 52 kann für alle technischen Replikate bzw. für alle bestimmten Wells 20 gleichartig oder für einzelne der technischen Replikate bzw. der bestimmten Wells 20 unterschiedlich ausführbar sein. Dadurch lassen sich beispielsweise Kontroll- und Testgruppen bilden.

Das wenigstens eine Modul 72 zur Analyse kann vor und/oder nach wenigstens einem zuvor beschriebenen Modul 70 zur Aufbereitung des Erhaltungsmedium 52 oder des oder der zu- und/oder abgeführten Expositionsmedien 52 angeordnet sein.

Ein Modul 72 kann zur physikalisch/chemischen oder biologischen Analyse innerhalb oder unterhalb der Standard-Multiwellplatte 18 ausführbar sein.

Eine direkte Analyse des biologischen Testsystems 12 in der Standard-Multiwellplatte 18 beispielsweise mittels fluoreszenzspektroskopischer Methoden kann ebenfalls vorgesehen sein.

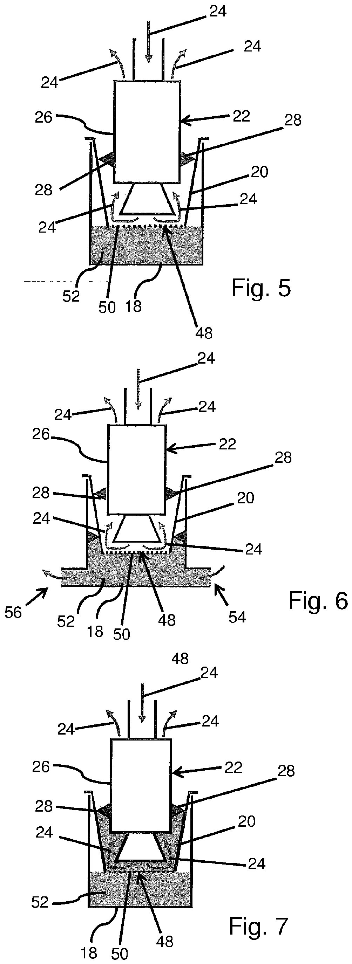

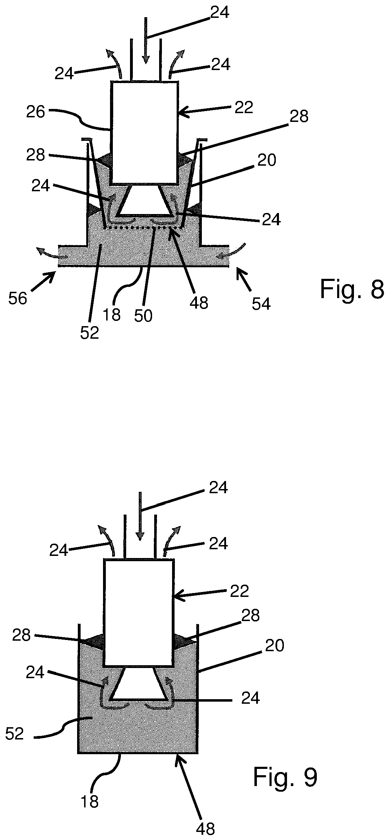

Fig. 5 bis 9 zeigen fünf unterschiedliche Anwendungs- bzw. Ausführungsbeispiele des erfindungsgemäßen Expositionsapparats in Verbindung einer Standard-Multiwellplatte (Fig. 5, 7 und 9) bzw. einer standard-multiwellplattenkompatiblen Expositionsvorrichtung (Fig. 6 und 8). Letztgenannte Expositionsvorrichtung ist in der DE 10 2007 030 413 A1 offenbart, deren Offenbarungsgehalt zur genannten Expositionsvorrichtung hiermit durch ausdrücklichen Verweis in die Offenbarung der vorliegenden Patentanmeldung aufgenommen wird.

Die Anwendungs- bzw. Ausführungsbeispiele unterscheiden sich in der Art des Expositionsmediums 24 (Fluidphase I: flüssig/gasförmig), in der Art der Zuführung des Erhaltungsmediums 52 (Fluidphase II: perfusiv/statisch) und - wie schon erwähnt - in der Art der Multiwellplatte (Standard-Multiwellplatte/ standard-multiwellplattenkompatible Expositionsvorrichtung gemäß DE 10 2007 030 413 A1).

Tabellarisch lassen sich die Anwendungs- bzw. Ausführungsbeispiele gemäß Fig. 5 bis 9 wie folgt aufgliedern:

| **Fig.** | **Phase I** | **Phase II** | **Aufbau** |
|---|---|---|---|
| **5** | Gas/Aerosol perfusiv | Flüssigkeit statisch | Barrierekultur (ALI) in Standard-Multiwellplatten |
| **6** | Gas/Aerosol perfusiv | Flüssigkeit perfusiv | Barrierekultur (ALI) in multiwellplattenkompatibler Expositionsvorrichtung gemäß DE 10 2007 030 413 A1 |
| **7** | Flüssigkeit perfusiv | Flüssigkeit statisch | Barrierekultur (liquid) in Standard-Multiwellplatten |
| **8** | Flüssigkeit perfusiv | Flüssigkeit perfusiv | Barrierekultur (liquid) in multiwellplattenkompatibler Expositionsvorrichtung gemäß DE 10 2007 030 413 A1 |
| **9** | Flüssigkeit perfusiv | - | Adhärent, submers in Standard-Multiwellplatten |

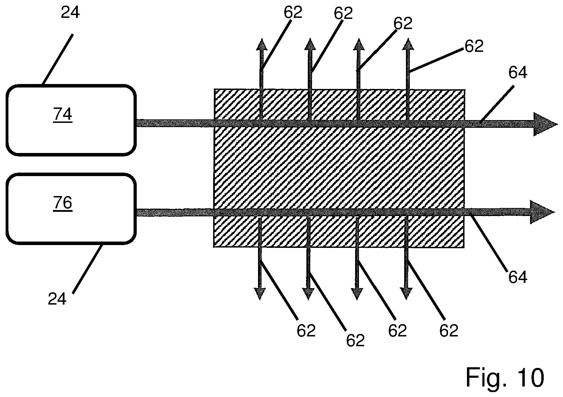

Fig. 10 zeigt schematisch eine mögliche Aufteilung des Expositionsmediums auf technische Replikate, die jeweils biologischen Testsystemen zugeordnet sind. Die Aufteilung erfolgt hierbei aus zwei unterschiedlichen Expositionsmedien 24 erfolgen, wobei das eine Expositionsmedium für eine Negativkontrolle aus Reinluft 74 besteht und das andere Expositionsmedium die eigentliche Probe bzw. Testsubstanz umfasst, beispielsweise in Form eines pharmakologisch wirksamen Aerosols 76.

Neben den jeweils den technischen Replikaten bzw. bestimmten Wells 20 zu- und aus diesen abgeleiteten Expositionsvolumenströmen 62 sind zwei weiterer Volumenströme 64 vorgesehen, über die das eine und das andere Expositionsmedium 74, 76 nach Art eine Vorförderung durch einen oberen Teil des Expositionsapparat 10 führbar ist, wobei vorliegend jeweils fünf Expositionsvolumenströme 62 aus jedem dieser Volumenströme 64 leitbar bzw. speisbar sind.

Der jeweilige weitere Volumenstrom 64 ist nach Art einer Vorförderung mit einer anderen Volumenflussrate, vorzugsweise größeren Volumenflussrate, betreibbar als die Summe der mit diesem weiteren Volumenstrom 64 in Verbindung stehenden einzelnen Expositionsvolumenströme 62.

Vorliegend ist fünf technischen Replikaten bzw. den entsprechenden Wells 20 je ein hier nicht dargestelltes Leitungselement 22 zugeordnet, durch das das Expositionsmedium 24 ohne Testsubstanz, beispielsweise als Reinluft 74, dieser Gruppe von Wells 20 zuleitbar und als so genannte Blindprobe im in-vitro-Experiment mitführbar ist.

Weiteren fünf technischen Replikaten bzw. den entsprechenden Wells 20 ist vorliegend je ein weiteres hier nicht dargestelltes Leitungselement 22 zugeordnet, durch das das Expositionsmedium 24 mit Testsubstanz, beispielsweise als pharmakologisch wirksames Aerosol 76, dieser Gruppe von Wells 20 zuleitbar und als eigentliche Probe im in-vitro-Experiment mitführbar ist.

Die Vorförderung bzw. der entsprechende weitern Volumenstrom 64 kann beispielsweise mit einem Volumenfluss von 500 ml/min betrieben werden, während jeder einzelne Expositionsvolumenstrom 66 mit einem Volumenfluss von beispielsweise 5 ml/min betrieben werden kann.

Jeder Expositionsvolumenstrom 64 ist darüber hinaus auch individuell mit einem vorgebbaren Volumenfluss für jedes Well einstellbar.

### Bezugszeichenliste

### (ist Teil der Beschreibung)

- 10: Expositionsapparat
- 12: biologisches Testsystem
- 14: Expositionsatmosphäre
- 16: Expositionsaufsatz
- 18: Standard-Multiwellplatte
- 20: Well
- 22: Leitungselement
- 24: Expositionsmedium
- 26: Außenseite des Leitungselements 22
- 28: Dichtmittel des Leitungselements 22
- 30: Innenwand des Wells 20
- 32: Basisaufnahme
- 34: Aufsatzhalterahmen
- 36: Verriegelungselement
- 38: Positionierhilfe
- 40: Stift
- 42: Aussparung
- 44: Aufsatzkopf
- 46: Verriegelungselement
- 48: Boden des Wells
- 50: permeabler Träger
- 52: Erhaltungsmedium
- 54: Zuleitung
- 56: Ableitung
- 58: Dichtmittel zwischen 34 und 44
- 60: Dichtmittel zwischen 16 und 18
- 62: Expositionsvolumenstrom
- 64: weiterer Volumenstrom
- 66: Aufbereitung Expositionsmedium
- 68: Detektion Expositionsmedium
- 70: Aufbereitung Erhaltungsmedium
- 72: Detektion Erhaltungsmedium

## Patentansprüche

1. Expositionsapparat (10) zur Durchführung von in-vitro-Experimenten mit technischen Replikaten wenigstens eines biologischen Testsystems (12), die perfusiv unter Ausbildung einer Expositionsatmosphäre (14) oberhalb des biologischen Testsystems (12) mit einem Expositionsmedium (24) beaufschlagbar sind, umfassend
• einen Expositionsaufsatz (16), der derart dimensioniert ist, dass dieser auf eine Standard-Multiwellplatte (18) aufsetzbar und mit dieser (18) mittels geeigneter Verbindungselemente (36) verbindbar ist, wobei der Expositionsaufsatz (16) mehrere Leitungselemente (22) mit jeweils zumindest einer Zuleitung (54) und zumindest einer Ableitung (56) für ein Expositionsmedium (24) aufweist,
• wobei jedes Leitungselement (22) aus der zur Standard-Multiwellplatte (18) weisenden Seite des Expositionsaufsatzes (16) hervorragt und einem bestimmtenWell (20) der Standard-Multiwellplatte (18) zur Ausbildung der Expositionsatmosphäre (14) innerhalb des bestimmten Wells (20) zugeordnet ist,
• wobei die Wells Aufnahmen sind, die bereits in der Standard-Multiwellplatte integriert sind, oder solche, die als separate Kulturgefäße, so genannte Inserts, in vorhandene integrierte Wells oder in entsprechend angeordnete Aussparungen der Standard-Multiwellplatte aufgenommen sind,
• wobei der Expositionsaufsatz (16) einen auf die Standard-Multiwellplatte (18) aufsetzbaren und mit dieser mittels der Verbindungselemente (36) verbindbaren Aufsatzhalterahmen (34) sowie einen bewegbaren auf dem Aufsatzhalterahmen (34) angeordneten und mit diesem (34) verbundenen Aufsatzkopf (44) aufweist,
• wobei das jeweils einem bestimmten Well (20) zugeordnete Leitungselement (22) nach dem Aufsetzen des Expositionsaufsatzes (16) auf die Standard-Multiwellplatte (18) durch Bewegen des mit den Leitungselementen (22) zusammenwirkenden Aufsatzkopfes (44) in Richtung Aufsatzhalterahmen (34) und Standard-Multiwellplatte (18) in das bestimmte Well (20) bewegbar und derart einbringbar ist, dass ein auf der Außenseite (26) des Leitungselements (22) vorgesehenes und dieses umlaufende Dichtmittel (28) das Leitungselement (22) gegenüber der Innenwand (30) des Wells (20) zur Ausbildung einer während des in-vitro-Experiments innerhalb jedes bestimmten Wells (20) abgeschlossenen Expositionsatmosphäre (14) dicht abschließt,
• wobei nur über das jeweils zugeordnete Leitungselement (22) Expositionsmedium (14) zu- und ableitbar ist, wobei der Aufsatzkopf (14) mittels vorzugsweise am Aufsatzkopf (44) angeordneter Ver- und Entriegelungselemente (46) mit dem Aufsatzhalterahmen (34) zur oder während der Durchführung des Experiments derart verriegelbar ist, dass die Leitungselemente (22) festgelegt sind, wobei die Verriegelungselemente (46) den Aufsatzkopf (44) und den Aufsatzhalterahmen (34) vorzugsweise unter Spannung zusammenhalten und wobei der Aufsatzkopf (44) nach der Durchführung des Experiments mittels der Ver- und Entriegelungselemente (46) von dem Aufsatzhalterahmen (34) entriegelbar ist, derart, dass die Leitungselemente (22) samt umlaufende Dichtmittel (28) mit Bewegen des Aufsatzkopfes (44) in entgegengesetzter Richtung zur vorherigen aus den Wells (20) lösbar sind.

2. Expositionsapparat (10) nach Anspruch 1, **dadurch gekennzeichnet, dass** zum dichten Abschließen der Expositionsatmosphäre (14) mehr als ein Dichtmittel (28, 60) zwischen Expositionsaufsatz (16) und Standard-Multiwellplatte (18) vorgesehen ist.

3. Expositionsapparat (10) nach Anspruch 2, **dadurch gekennzeichnet, dass** das Dichtmittel (28, 60) eine das jeweilige Well (20) oder die Öffnung des jeweiligen Wells (20) umlaufende Dichtung ist.

4. Expositionsapparat (10) nach Anspruch 2 oder 3, **dadurch gekennzeichnet, dass** das Dichtmittel (60) eine auf die Standard-Multiwellplatte (18) aufbringbare oder aufgebrachte und/oder an der zur Standard-Multiwellplatte (18) weisenden Seite des Expositionsaufsatzes (16) anbringbare oder angebrachte Dichtmatte mit entsprechenden Aussparungen für die bestimmten Wells (20) oder Öffnungen der bestimmten Wells (20) und/oder mit entsprechenden Aussparungen für die den bestimmten Wells (20) zugeordneten Leitungselementen (22) ist.

5. Expositionsapparat (10) nach einem der Ansprüche 1 bis 4, **dadurch kennzeichnet, dass** eine dem Multiwellplattenstandard kompatible Multiwellplatte an Stelle der Standard-Multiwellplatte einsetzbar ist.

6. Expositionsapparat (10) nach einem der Ansprüche 1 bis 5, **dadurch kennzeichnet, dass** die Verbindungselemente derart sind, dass der Expositionsaufsatz (16) auf der Standard-Multiwellplatte (18) reversibel festklemmbar ist.

7. Expositionsapparat (10) nach einem der Ansprüche 1 bis 6, **dadurch kennzeichnet, dass** die Verbindungselemente Verriegelungselemente (36) umfassen, welche den Expositionsaufsatz (16) und die Standard-Multiwellplatte (18) vorzugsweise unter Spannung zusammenhalten.

8. Expositionsapparat (10) nach einem der Ansprüche 1 bis 7, **dadurch gekennzeichnet, dass** die Verbindungselemente derart sind, dass die Standard-Multiwellplatte (18) und vorzugsweise die bestimmten Wells (20) während der Durchführung des Experiments festgelegt sind.

9. Expositionsapparat (10) nach einem der Ansprüche 1 bis 8, **dadurch gekennzeichnet, dass** es sich bei den Verbindungselementen um am Expositionsaufsatz (16) angeordnete Klemmbacken handelt.

10. Expositionsapparat (10) nach Anspruch 1, **dadurch gekennzeichnet, dass** der Aufsatzhalterahmen (34) die Standard-Multiwellplatte (18) zumindest teilweise umhüllt.

11. Expositionsapparat (10) nach Anspruch 1, **dadurch gekennzeichnet, dass** zur Durchführung des Experiments zunächst der Aufsatzhalterahmen (34) mit der Standard-Multiwellplatte (18) und anschließend der Aufsatzkopf (44) mit dem Aufsatzhalterahmen (34) verriegelbar ist und dass nach der Durchführung des Experiments zunächst der Aufsatzkopf (44) vom Aufsatzhalterahmen (34) entriegelbar und anschließend der Aufsatzhalterahmen (34) von der die Standard-Multiwellplatte (18) entriegelbar ist.

12. Expositionsapparat (10) nach einem der Ansprüche 1 bis 11, **dadurch gekennzeichnet, dass** derselbe (10) eine Basisaufnahme (32) zur Aufnahme der Standard-Multiwellplatte (18) aufweist.

13. Expositionsapparat (10) nach Anspruch 12, **dadurch gekennzeichnet, dass** die Basisaufnahme (32) die Standard-Multiwellplatte (18) zumindest teilweise umhüllt.

14. Expositionsapparat (10) nach Anspruch 12 oder 13, **dadurch gekennzeichnet, dass** der Expositionsaufsatz (16), insbesondere der Aufsatzhalterahmen (34) des Expositionsaufsatzes (16), mittels wenigstens einer Positionierhilfe (38) auf der Basisaufnahme (32) positionierbar und aufsetzbar ist.

15. Expositionsapparat (10) nach Anspruch 14, **dadurch gekennzeichnet, dass** die Positionierhilfe (38) einen auf der Basisaufnahme (32) angeordneten Stift (40) und eine für diesen (40) entsprechend vorgesehene Aussparung (42) im Expositionsaufsatz (16), insbesondere im Aufsatzhalterahmen (34) des Expositionsaufsatzes (16), aufweist.

16. Expositionsapparat (10) nach einem Ansprüche 12 bis 15, **dadurch gekennzeichnet, dass** die in der Basisaufnahme (32) aufgenommene Standard-Multiwellplatte (18) von der Basisaufnahme (32) und dem auf der Standard-Multiwellplatte (18) und der Basisaufnahme (32) aufgesetzten Expositionsaufsatz (16) oder Aufsatzhalterahmen (34) des Expositionsaufsatzes (16) nach dem Verriegeln vollständig umhüllt ist.

17. Expositionsapparat (10) nach einem der Ansprüche 1 bis 16, **dadurch gekennzeichnet, dass** zwischen Aufsatzhalterahmen (34) und Aufsatzkopf (44) ein das Leitungselement (22) umlaufendes Dichtmittel (58) vorgesehen ist.

18. Expositionsapparat (10) nach zumindest einem der vorherigen Ansprüche, **dadurch gekennzeichnet, dass** der Expositionsaufsatz (16), der Aufsatzkopf (44), der Aufsatzhalterahmen (34) und/oder die Basisaufnahme (32) ganz oder teilweise, jeweils für sich oder zumindest teilweise zusammen temperierbar, insbesondere elektrisch beheizbar sind, vorzugsweise derart, dass sich innerhalb des Expositionsapparats (10) ein thermischer Gradient ausbildet, der insbesondere zur gezielten Abscheidung von Substanzen aus einem Aerosolstrom mittels Thermophorese nutzbar ist.

## Claims

1. Exposure apparatus (10) for carrying out in vitro experiments with technical replicates of at least one biological test system (12), to which an exposure medium (24) can be applied by perfusion, forming an exposure atmosphere (14) above the biological test system (12), comprising
• an exposure attachment (16), which is dimensioned in such a way that this can be mounted on a standard multiwell plate (18) and connected to the latter (18) by means of suitable connecting elements (36), wherein the exposure attachment (16) has multiple line elements (22) each having at least one feed line (54) and at least one discharge line (56) for an exposure medium (24),
• wherein each line element (22) projects out of the side of the exposure attachment (16) facing the standard multiwell plate (18) and is assigned to a specific well (20) of the standard multiwell plate (18), forming the exposure atmosphere (14) within the specific well (20),
• wherein the wells are receptacles which are already integrated in the standard multiwell plate or those which are accommodated, as separate culture vessels, so-called inserts, in existing integrated wells or in appropriately arranged recesses in the standard multiwell plate,
• wherein the exposure attachment (16) has an attachment holding frame (34) that can be placed on the standard multiwell plate (18) and connected to the latter by means of the connecting elements (36), and also an attachment head (44) that is movably arranged on the attachment holding frame (34) and connected to the latter (34),
• wherein, following the placement of the exposure attachment (16) on the standard multiwell plate (18), the line element (22) respectively assigned to a specific well (20) can be moved into the specific well (20) by moving the attachment head (14) interacting with the line elements (22) in the direction of the attachment holding frame (34) and standard multiwell plate (18), and can be inserted in such a way that a sealing means (28) provided on the outside (26) of the line element (22) and running around the latter seals the line element (22) tightly against the inner wall (30) of the well (20) to form an exposure atmosphere (14) that is sealed off during the in vitro experiment within each specific well (20),
• wherein exposure medium (14) can be supplied and discharged only via the respectively associated line element (22), wherein, for or during the performance of the experiment, the attachment head (14) can be locked to the attachment holding frame (34) by means of locking and unlocking elements (46) preferably arranged on the attachment head (44) in such a way that the line elements (22) are fixed, wherein the locking elements (46) hold the attachment head (44) and the attachment holding frame (34) together, preferably under tension, and wherein, following the performance of the experiment, the attachment head (44) can be unlocked from the attachment holding frame (34) by means of the locking and unlocking elements (46), in such a way that the line elements (22) together with the circumferential sealing means (28) can be detached from the wells (20) with a movement of the attachment head (44) in the opposite direction to the previous.

2. Exposure apparatus (10) according to Claim 1, **characterized in that** for the tight sealing of the exposure atmosphere (14), more than one sealing means (28, 60) is provided between exposure attachment (16) and standard multiwell plate (18).

3. Exposure apparatus (10) according to Claim 2, **characterized in that** the sealing means (28, 60) is a seal running around the respective well (20) or the opening of the respective well (20).

4. Exposure apparatus (10) according to Claim 2 or 3, **characterized in that** the sealing means (60) is a sealing mat that can be or is applied to the standard multiwell plate (18) and/or can be or is applied to the side of the exposure attachment (16) facing the standard multiwell plate (18), and has appropriate recesses for the specific wells (20) or openings of the specific wells (20) and/or has appropriate recesses for the line elements (22) assigned to the specific wells (20).

5. Exposure apparatus (10) according to one of Claims 1 to 4, **characterized in that** a multiwell plate that is compatible with the multiwell plate standard can be used instead of the standard multiwell plate.

6. Exposure apparatus (10) according to one of Claims 1 to 5, **characterized in that** the connecting elements are such that the exposure attachment (16) can be firmly clamped reversibly to the standard multiwell plate (18).

7. Exposure apparatus (10) according to one of Claims 1 to 6, **characterized in that** the connecting elements comprise locking elements (36), which hold the exposure attachment (16) and the standard multiwell plate (18) together, preferably under tension.

8. Exposure apparatus (10) according to one of Claims 1 to 7, **characterized in that** the connecting elements are such that the standard multiwell plate (18) and preferably the specific wells (20) are fixed during the performance of the experiment.

9. Exposure apparatus (10) according to one of Claims 1 to 8, **characterized in that** the connecting elements are clamping jaws arranged on the exposure attachment (16).

10. Exposure apparatus (10) according to Claim 1, **characterized in that** the attachment holding frame (34) at least partly encloses the standard multiwell plate (18).

11. Exposure apparatus (10) according to Claim 1, **characterized in that** to perform the experiment, firstly the attachment holding frame (34) can be locked to the standard multiwell plate (18) and then the attachment head (44) can be locked to the attachment holding frame (34) and **in that**, following the performance of the experiment, firstly the attachment head (44) can be unlocked from the attachment holding frame (34) and then the attachment holding frame (34) can be unlocked from the standard multiwell plate (18).

12. Exposure apparatus (10) according to one of Claims 1 to 11, **characterized in that** the same (10) has a base receptacle (32) to receive the standard multiwell plate (18).

13. Exposure apparatus (10) according to Claim 12, **characterized in that** the base receptacle (32) at least partly encloses the standard multiwell plate (18) .

14. Exposure apparatus (10) according to Claim 12 or 13, **characterized in that** the exposure attachment (16), in particular the attachment holding frame (34) of the exposure attachment (16), can be positioned and placed on the base receptacle (32) by means of at least one positioning aid (38).

15. Exposure apparatus (10) according to Claim 14, **characterized in that** the positioning aid (38) has a pin (40) arranged on the base receptacle (32) and a recess (42) correspondingly provided for the same (40) in the exposure attachment (16), in particular in the attachment holding frame (34) of the exposure attachment (16).

16. Exposure apparatus (10) according to one of Claims 12 to 15, **characterized in that,** following the locking, the standard multiwell plate (18) accommodated in the base receptacle (32) is completely enclosed by the base receptacle (32) and the exposure attachment (16) or attachment holding frame (34) of the exposure attachment (16) that is placed on the standard multiwell plate (18) and the base receptacle (32).

17. Exposure apparatus (10) according to one of Claims 1 to 16, **characterized in that** a sealing means (58) running around the line element (22) is provided between attachment holding frame (34) and attachment head (44).

18. Exposure apparatus (10) according to at least one of the preceding claims, **characterized in that** the exposure attachment (16), the attachment head (44), the attachment holding frame (34) and/or the base receptacle (32) can be temperature-controlled, wholly or partly, each on its own or at least partly together, in particular can be heated electrically, preferably in such a way that within the exposure apparatus (10) there is formed a thermal gradient which, in particular, can be utilized for the specific deposition of substances from an aerosol stream by means of thermophoresis.

## Revendications

1. Appareil d'exposition (10) permettant de réaliser des expériences in vitro avec des réplicats techniques d'au moins un système d'essai biologique (12), lesquels peuvent être exposés à un milieu d'exposition (24) par perfusion avec formation d'une atmosphère d'exposition (14) au-dessus du système d'essai biologique (12), comportant
- un élément rapporté d'exposition (16) qui est dimensionné de telle sorte que celui-ci peut être placé sur une plaque Multiwell standard (18) et peut être relié à celle-ci (18) au moyen d'éléments de liaison appropriés (36), l'élément rapporté d'exposition (16) comprenant plusieurs éléments de conduite (22) dotés respectivement d'au moins une conduite d'amenée (54) et d'au moins une conduite d'évacuation (56) pour un milieu d'exposition (24),
- chaque élément de conduite (22) faisant saillie à partir du côté, tourné vers la plaque Multiwell standard (18), de l'élément rapporté d'exposition (16) et étant associé à un puits déterminé (20) de la plaque Multiwell standard (18) pour former l'atmosphère d'exposition (14) à l'intérieur du puits déterminé (20),
- les puits étant des logements qui sont déjà intégrés dans la plaque Multiwell standard ou qui, en tant que récipients de culture séparés, dits inserts, sont logés dans des puits intégrés présents ou dans des évidements disposés de manière correspondante de la plaque Multiwell standard,
- l'élément rapporté d'exposition (16) comprenant un cadre de retenue d'élément rapporté (34) pouvant être placé sur la plaque Multiwell standard (18) et pouvant être relié à celle-ci au moyen des éléments de liaison (36) ainsi qu'une tête d'élément rapporté (44) disposée de manière mobile sur le cadre de retenue d'élément rapporté (34) et reliée à celui-ci (34),
- l'élément de conduite (22) associé respectivement à un puits déterminé (20) pouvant être déplacé et introduit dans le puits déterminé (20), après le placement de l'élément rapporté d'exposition (16) sur la plaque Multiwell standard (18) par déplacement de la tête d'élément rapporté (44) coopérant avec les éléments de conduite (22) en direction du cadre de retenue d'élément rapporté (34) et de la plaque Multiwell standard (18), de telle sorte qu'un moyen d'étanchéité (28) prévu sur le côté extérieur (26) de l'élément de conduite (22) et entourant celui-ci ferme hermétiquement l'élément de conduite (22) par rapport à la paroi interne (30) du puits (20) pour former une atmosphère d'exposition (14) fermée à l'intérieur de chaque puits déterminé (20) pendant l'expérience in vitro,
- le milieu d'exposition (14) ne pouvant être amené et évacué que par le biais de l'élément de conduite (22) respectivement associé, la tête d'élément rapporté (14) pouvant être verrouillée, au moyen d'éléments de verrouillage et de déverrouillage (46) disposés de préférence sur la tête d'élément rapporté (44), avec le cadre de retenue d'élément rapporté (34) pour réaliser l'expérience ou pendant la réalisation de celle-ci, de telle sorte que les éléments de conduite (22) soient fixés, les éléments de verrouillage (46) maintenant ensemble la tête d'élément rapporté (44) et le cadre de retenue d'élément rapporté (34) de préférence sous contrainte, et la tête d'élément rapporté (44) pouvant être déverrouillée du cadre de retenue d'élément rapporté (34) au moyen des éléments de verrouillage et de déverrouillage (46) après que l'expérience a été réalisée, de telle sorte que les éléments de conduite (22), y compris les moyens d'étanchéité périphériques (28), puissent être détachés des puits (20) par déplacement de la tête d'élément rapporté (44) en sens inverse au sens précédent.

2. Appareil d'exposition (10) selon la revendication 1, **caractérisé en ce que**, pour fermer hermétiquement l'atmosphère d'exposition (14), plus d'un moyen d'étanchéité (28, 60) est prévu entre l'élément rapporté d'exposition (16) et la plaque Multiwell standard (18).

3. Appareil d'exposition (10) selon la revendication 2, **caractérisé en ce que** le moyen d'étanchéité (28, 60) est un joint d'étanchéité entourant le puits respectif (20) ou l'ouverture du puits respectif (20).

4. Appareil d'exposition (10) selon la revendication 2 ou 3, **caractérisé en ce que** le moyen d'étanchéité (60) est un mat d'étanchéité pouvant être appliqué ou appliqué sur la plaque Multiwell standard (18) et/ou pouvant être appliqué ou appliqué sur le côté, tourné vers la plaque Multiwell standard (18), de l'élément rapporté d'exposition (16), lequel mat d'étanchéité est doté d'évidements correspondants pour les puits déterminés (20) ou des ouvertures des puits déterminés (20) et/ou est doté d'évidements correspondants pour les éléments de conduite (22) associés aux puits déterminés (20).

5. Appareil d'exposition (10) selon l'une quelconque des revendications 1 à 4, **caractérisé en ce qu'**une plaque Multiwell compatible avec la norme de plaque Multiwell peut être utilisée au lieu de la plaque Multiwell standard.

6. Appareil d'exposition (10) selon l'une quelconque des revendications 1 à 5, **caractérisé en ce que** les éléments de liaison sont tels que l'élément rapporté d'exposition (16) peut être serré fixement de manière réversible sur la plaque Multiwell standard (18).

7. Appareil d'exposition (10) selon l'une quelconque des revendications 1 à 6, **caractérisé en ce que** les éléments de liaison comportent des éléments de verrouillage (36) qui maintiennent ensemble l'élément rapporté d'exposition (16) et la plaque Multiwell standard (18) de préférence sous contrainte.

8. Appareil d'exposition (10) selon l'une quelconque des revendications 1 à 7, **caractérisé en ce que** les éléments de liaison sont tels que la plaque Multiwell standard (18) et de préférence les puits déterminés (20) sont fixés pendant la réalisation de l'expérience.

9. Appareil d'exposition (10) selon l'une quelconque des revendications 1 à 8, **caractérisé en ce que** les éléments de liaison sont des mâchoires de serrage disposées sur l'élément rapporté d'exposition (16).

10. Appareil d'exposition (10) selon la revendication 1, **caractérisé en ce que** le cadre de retenue d'élément rapporté (34) enveloppe au moins partiellement la plaque Multiwell standard (18).

11. Appareil d'exposition (10) selon la revendication 1, **caractérisé en ce que**, pour réaliser l'expérience, tout d'abord le cadre de retenue d'élément rapporté (34) peut être verrouillé avec la plaque Multiwell standard (18) et ensuite la tête d'élément rapporté (44) peut être verrouillée avec le cadre de retenue d'élément rapporté (34), et **en ce qu'**après la réalisation de l'expérience, tout d'abord la tête d'élément rapporté (44) peut être déverrouillée du cadre de retenue d'élément rapporté (34) et ensuite le cadre de retenue d'élément rapporté (34) peut être déverrouillé de la plaque Multiwell standard (18).

12. Appareil d'exposition (10) selon l'une quelconque des revendications 1 à 11, **caractérisé en ce que** celui-ci (10) comprend un logement de base (32) servant à loger la plaque Multiwell standard (18).

13. Appareil d'exposition (10) selon la revendication 12, **caractérisé en ce que** le logement de base (32) enveloppe au moins partiellement la plaque Multiwell standard (18).

14. Appareil d'exposition (10) selon la revendication 12 ou 13, **caractérisé en ce que** l'élément rapporté d'exposition (16), en particulier le cadre de retenue d'élément rapporté (34) de l'élément rapporté d'exposition (16), peut être positionné et peut être placé sur le logement de base (32) au moyen d'au moins un auxiliaire de positionnement (38) .

15. Appareil d'exposition (10) selon la revendication 14, **caractérisé en ce que** l'auxiliaire de positionnement (38) comprend une goupille (40) disposée sur le logement de base (32) et un évidement (42), prévu de manière correspondante pour celle-ci (40), dans l'élément rapporté d'exposition (16), en particulier dans le cadre de retenue (34) de l'élément rapporté d'exposition (16) .

16. Appareil d'exposition (10) selon l'une quelconque des revendications 12 à 15, **caractérisé en ce que** la plaque Multiwell standard (18) logée dans le logement de base (32) est enveloppée complètement par le logement de base (32) et l'élément rapporté d'exposition (16) ou le cadre de retenue (34) de l'élément rapporté d'exposition (16) placé sur la plaque Multiwell standard (18) et le logement de base (32) après le verrouillage.

17. Appareil d'exposition (10) selon l'une quelconque des revendications 1 à 16, **caractérisé en ce qu'**un moyen d'étanchéité (58) entourant l'élément de conduite (22) est prévu entre le cadre de retenue d'élément rapporté (34) et la tête d'élément rapporté (44).

18. Appareil d'exposition (10) selon au moins l'une quelconque des revendications précédentes, **caractérisé en ce que** l'élément rapporté d'exposition (16), la tête d'élément rapporté (44), le cadre de retenue d'élément rapporté (34) et/ou le logement de base (32) peuvent être thermorégulés, en particulier peuvent être chauffés électriquement, complètement ou partiellement, respectivement individuellement ou au moins partiellement conjointement, de préférence de telle sorte qu'un gradient thermique se forme à l'intérieur de l'appareil d'exposition (10), lequel gradient peut en particulier être utilisé pour la séparation ciblée, par thermophorèse, de substances à partir d'un flux d'aérosol.
